(19) Europäisches Patentamt
European Patent Office
Office européen des brevets

(11) EP 1 590 337 B1

(12) FASCICULE DE BREVET EUROPEEN

(45) Date de publication et mention de la délivrance du brevet:
**13.08.2008 Bulletin 2008/33**

(21) Numéro de dépôt: **04704255.1**

(22) Date de dépôt: **22.01.2004**

(51) Int Cl.:
***C07D 277/46*** *(2006.01)* ***C07D 417/12*** *(2006.01)*
***A61K 31/426*** *(2006.01)* ***A61K 31/427*** *(2006.01)*
***A61K 31/4422*** *(2006.01)* ***A61P 25/28*** *(2006.01)*
***A61P 25/16*** *(2006.01)*

(86) Numéro de dépôt international:
**PCT/FR2004/000140**

(87) Numéro de publication internationale:
**WO 2004/067520 (12.08.2004 Gazette 2004/33)**

(54) **DERIVES D' ACYLAMINOTHIAZOLE, LEUR PREPARATION ET LEUR UTILISATION EN TANT QU' INHIBITEURS DE LA PRODUCTION DU PEPTIDE BETA-AMYLOIDE**

ACYLAMINOTHIAZOLDERIVATE, VERFAHREN ZU DEREN HERSTELLUNG UND DEREN VERWENDUNG ALS INHIBITOREN DER PRODUKTION VON BETA-AMYLOIDPEPTID

ACYLAMINOTHIAZOLE DERIVATIVES, PREPARATION METHOD THEREOF AND USE OF SAME AS BETA-AMYLOID PEPTIDE PRODUCTION INHIBITORS

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **23.01.2003 FR 0300703**

(43) Date de publication de la demande:
**02.11.2005 Bulletin 2005/44**

(73) Titulaire: **Sanofi-Aventis**
**75013 Paris (FR)**

(72) Inventeurs:
- **DESPEYROUX, Pierre**
  **F-31860 Labarthe sur Leze (FR)**
- **FREHEL, Daniel**
  **F-31160 Estadens (FR)**
- **SCHOENTJES, Bruno**
  **F-76230 Bois-Guillaume (FR)**
- **VAN DORSSELAER, Viviane**
  **F-67100 Strasbourg (FR)**

(74) Mandataire: **Kugel, Dominique et al**
**Sanofi-Aventis**
**174 avenue de France**
**75013 Paris (FR)**

(56) Documents cités:
**WO-A-98/28268** **WO-A-03/014095**

- **DATABASE CA [Online] CHEMICAL ABSTRACTS SERVICE, COLUMBUS, OHIO, US; KOJIMA ET AL.: XP002193188 extrait de STN Database accession no. 132:322142 & WO 00/24392 A (SUMITOMO PHARMACEUTICALS) 4 mai 2000 (2000-05-04)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet des dérivés d'acylaminothiazole leur préparation et leur utilisation en thérapeutique.

L'invention a pour premier objet des composés répondant à la formule générale (I) :

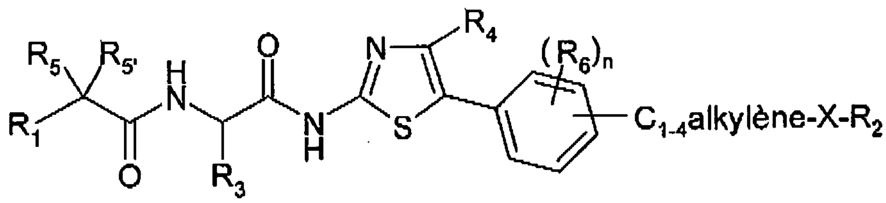

(I)

dans laquelle,
X représente un atome d'oxygène ou de soufre ;

$R_1$ représente un groupe $C_{1-10}$ alkyle éventuellement substitué par un $C_{3-7}$ cycloalkyle, un phényle, un thiényle ; ou $R_1$ représente un groupe $C_{3-7}$ cycloalkyle, thiényle, pyridinyle, pyrimidinyle ;
les groupes thiényle étant éventuellement substitués par un à 3 groupes $C_{1-3}$ alkyle ; le groupe phényle étant éventuellement substitué par un à 5 atomes d'halogène ou groupes $C_{1-3}$ alkyle, $C_{1-3}$ alcoxy, $C_{1-3}$ fluoroalkyle, $C_{1-3}$ fluoroalcoxy ;
$R_2$ représente un groupe $C_{1-6}$ alkyle éventuellement substitué par un groupe $C_{3-7}$ cycloalkyle, phényle, $C_{1-3}$ alcoxy, hydroxy ; ou $R_2$ représente un groupe $C_{3-7}$ cycloalkyle, pipéridinyle, phényle ou pyridinyle ;
les groupes $C_{3-7}$ cycloalkyle et pipéridinyle étant éventuellement substitués par un ou plusieurs groupes $C_{1-3}$ alkyle, $C_{1-3}$ alcoxy, hydroxy, $C_{1-3}$ fluoroalkyle, $C_{1-3}$ fluoroalcoxy ;
les groupes phényle et pyridinyle étant éventuellement substitués par un ou plusieurs atomes d'halogène ou groupes $C_{1-3}$ alkyle, $C_{1-3}$ alcoxy, hydroxy, $C_{1-3}$ fluoroalkyle, $C_{1-3}$ fluoroalcoxy ;
$R_3$ représente un atome d'hydrogène ou un groupe $C_{1-6}$ alkyle éventuellement substitué par un groupe $C_{3-7}$ cycloalkyle ;
$R_4$ représente un atome d'hydrogène ou un groupe $C_{1-6}$ alkyle ;
$R_5$ et $R_{5'}$ représentent, indépendamment l'un de l'autre, un atome d'hydrogène, d'halogène, un groupe hydroxy, $C_{1-3}$ alkyle; ou $R_5$ et $R_{5'}$ forment ensemble un groupe oxo ou oxime tel que :

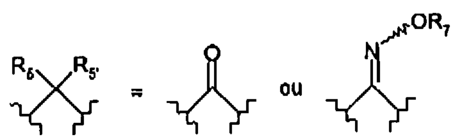

où $R_7$ représente un atome d'hydrogène ou un $C_{1-3}$ alkyle ;
n représente un nombre entier allant de 0 à 3 ; et
$R_6$ représente, indépendamment l'un de l'autre lorsque n = 2 ou 3, un atome d'hydrogène, d'halogène, un groupe hydroxy, $C_{1-3}$ alkyle, $C_{1-3}$ alcoxy, $C_{1-3}$ fluoroalkyle ou $C_{1-3}$ fluoroalcoxy.

WO 03/014095 et WO 98/28268 décrivent d'autres composés inhibiteurs de la production du peptide β-amyloïde, utiles dans le traitement de la maladie d'Alzheimer.

Parmi les composés de formule générale (I), un sous-groupe de composés préférés est constitué par les composés pour lesquels :

- X représente un atome d'oxygène ou de soufre; et/ou
- $R_1$ représente un groupe $C_{1-5}$ alkyle de préference un méthyle, éthyle, 1-méthyléthyle, 1,1-diméthyléthyle, propyle, 1-méthylpropyle, 2-méthylpropyle, 1-éthylpropyle, éventuellement substitué par un phényle, un thiényle ; ou $R_1$ représente un groupe $C_{3-7}$ cycloalkyle, de préférence un cyclohexyle, un groupe thiényle, pyridinyle ; les groupes thiényle étant éventuellement substitués par un ou deux groupes $C_{1-3}$ alkyle, de préférence méthyle ; le groupe phényle étant éventuellement substitué par un ou deux atomes d'halogène, de préférence chlore ou fluor ; et/ou

- $R_2$ représente un groupe $C_{1-6}$ alkyle, de préférence un éthyle, 1-méthyléthyle ; ou $R_2$ représente un groupe $C_{3-7}$ cycloalkyle, de préférence un cyclohexyle, phényle ou pyridinyle ;
  le groupe phényle étant éventuellement substitué par un à trois groupes $C_{1-3}$ alkyle, de préférence méthyle, éthyle, $C_{1-3}$ alcoxy, de préférence méthoxy, éthoxy, hydroxy, fluoroalcoxy, de préférence trifluorométhoxy, ou atomes d'halogène, de préférence chlore, fluor ; et/ou
- $R_3$ représente un groupe $C_{1-6}$ alkyle, de préférence un méthyle, éthyle, propyle; et/ou
- $R_4$ représente un atome d'hydrogène ou un groupe $C_{1-6}$ alkyle, de préférence un méthyle ou un 4-méthylpentyl ; et/ou
- $R_5$ et $R_{5'}$ représentent, indépendamment l'un de l'autre, un atome d'hydrogène, un hydroxy; ou $R_5$ et $R_{5'}$ forment ensemble un groupe oxo ; et/ou
- $R_6$ représente un atome d'hydrogène, d'halogène, de préférence un chlore ou un fluor, un $C_{1-3}$ alkyle, de préférence un méthyle, un $C_{1-3}$ alcoxy, de préférence un méthoxy ou un éthoxy ; et/ou
- n est égal à 0 ou 1.

Les composés pour lesquels à la fois X, $R_1$, $R_2$, $R_3$, $R_4$, $R_6$, $R_{5'}$, $R_6$ et n sont tels que définis ci-dessus dans le sous-groupe de composés préférés, sont particulièrement préférés et plus spécifiquement parmi ceux-ci les composés pour lesquels :

X représente un atome d'oxygène ; et/ou
le groupe $C_{1-4}$ alkylène est un méthylène ; et/ou
le carbone portant le groupe $R_3$ est de configuration (S).

L'invention a également pour objet, parmi les composés de formule générale (I), des composés pour lesquels :

X représente un atome d'oxygène ou de soufre ;
$R_1$ représente un groupe $C_{1-10}$ alkyle éventuellement substitué par un $C_{3-7}$ cycloalkyle, un phényle, un thiényle ; ou $R_1$ représente un groupe $C_{3-7}$ cycloalkyle, thiényle, pyridinyle, pyrimidinyle ;
les groupes thiényle étant éventuellement substitués par un à 3 groupes $C_{1-3}$ alkyle ; le groupe phényle étant éventuellement substitué par un à 5 atomes d'halogène ou groupes $C_{1-3}$ alkyle, $C_{1-3}$ alcoxy, $C_{1-3}$ fluoroalkyle, $C_{1-3}$ fluoroalcoxy ;
$R_2$ représente un groupe $C_{1-6}$ alkyle substitué par un groupe phényle ou $R_2$ représente un groupe phényle ou pyridinyle, lesdits groupes phényle et pyridinyle étant substitués par un ou plusieurs groupes CN, par exemple un à trois groupes CN ;
$R_3$ représente un atome d'hydrogène ou un groupe $C_{1-6}$ alkyle éventuellement substitué par un groupe $C_{3-7}$ cycloalkyle ;
$R_4$ représente un atome d'hydrogène ou un groupe $C_{1-6}$ alkyle ;
$R_5$ et $R_{5'}$ représentent, indépendamment l'un de l'autre, un atome d'hydrogène, d'halogène, un groupe hydroxy, $C_{1-3}$ alkyle; ou $R_5$ et $R_{5'}$ forment ensemble un groupe oxo ou oxime tel que :

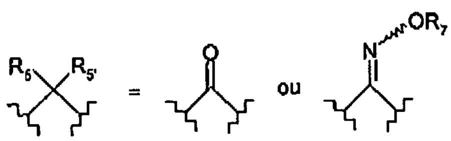

où $R_7$ représente un atome d'hydrogène ou un $C_{1-3}$ alkyle ;
n représente un nombre entier allant de 0 à 3 ; et
$R_6$ représente, indépendamment l'un de l'autre lorsque n = 2 ou 3, un atome d'hydrogène, d'halogène, un groupe hydroxy, $C_{1-3}$ alkyle, $C_{1-3}$ alcoxy, $C_{1-3}$ fluoroalkyle ou $C_{1-3}$ fluoroalcoxy.

A titre d'exemple de composés préférés, on peut citer les composés suivants :

**1.** (2*S*)-2-{[(2*R*)-2-cyclohexyl-2-hydroxyacétyl]amino}-*N*-(5-{2-[(cyclohoxyloxy)-méthyl]phényl}-1,3-thiazol-2-yl) pentanamide

**2.** (2*S*)-2-{[(2*S*)-2-cyclohexyl-2-hydroxyacétyl]amino}-*N*-(5-{2-[(cyclohexyloxy)-méthyl]phényl}-1,3-thiazol-2-yl) pentanamide

**3.** (2*S*)-*N*-(5-{2-[(cyclohexyloxy)méthyl]phényl}-1,3-thiazol-2-yl)-2-{[2-(3-pyridinyl)acétyl]amino}pentanamide

**4.** *N*-((1*S*)-1-{[(5-{2-[(cyclohexyloxy)méthyl]phényl}-1,3-thiazol-2-yl)amino]-carbonyl}butyl)-2-hydroxy-4-méthylpentanamide

**5.** (2*S*)-*N*-{5-[2-(isopropoxyméthyl)phényl]-4-méthyl-1,3-thiazol-2-yl}-2-{[2-(3-thiényl)acétyl]amino}pentanamide

**6.** (2*S*)-*N*-{5-[2-(isopropoxyméthyl)phényl]-1,3-thiazol-2-yl}-2-{[2-(3-thiényl)-acétyl]amino}pentanamide

**7.** (2*S*)-*N*-{5-[2-(isopropoxyméthyl)phényl]-1,3-thiazol-2-yl}-2-{[3-(3-thiényl)-propanoyl]amino}pentanamide

**8.** (2*S*)-*N*-{5-[2-(phénoxyméthyl)phényl]-1,3-thiazol-2-yl}-2-{[2-(3-thiényl)-acétyl]amino}pentanamide

**9.** (2*S*)-2-{[(2*S*)-2-hydroxy-3,3-diméthylbutanoyl]amino}-*N*-{5-[2-(phénoxy-méthyl)phényl]-1,3-thiazol-2-yl}pentanamide

**10.** (2*S*)- *N*-{5-(2-{phénoxyméthyl)phényl]-1,3-thiazol-2-yl}-2-{[2-(2-thiényl)acétyl]-amino}butanamide

**11.** (2*S*)-2-{[(2*S*)-2-hydroxy-3,3-diméthylbutanoyl]amino}-*N*-{5-[2-(isopropoxy-méthyl)phényl]-1,3-thiazol-2-yl}pentanamide

**12.** (2*S*)- *N*-{5-[2-(isopropoxyméthyl)phényl]-1,3-thiazol-2-yl}-2-{[2-(2-thiényl)-acétyl]amino}pentanamide

**13.** (2*S*)-2-[(3,3-diméthylbutanoyl)amino]-*N*-{5-[2-(issopropoxyméthyl)phényl]-1,3-thiazol-2-yl}pentanamide

**14.** *N*-{(1*S*)-1-[({5-[2-(isopropoxyméthyl)phényl]-1,3-thiazol-2-yl}-amino)carbonyl]-butyl}-3-méthyl-2-oxopentanamide

**15.** (2*S*)-*N*-{5-[2-(éthoxyméthyl)phényl]-1,3-thiazol-2-yl}-2-{[(2*S*)-2-hydroxy-3,3-diméthylbutanoyl]amino}pentanamide

**30.** (2*S*)-*N*-(5-{2-[(3,4-diméthoxyphénoxy)méthyl]phényl}-1,3-thiazol-2-yl)-2-{[(2*S*)-2-hydroxy-3,3-diméthylbutanoyl]amino}pentanamide

**31.** (2*S*)-*N*-(5-{2-[(2,6-diméthoxyphénoxy)méthyl]phényl}-1,3-thiazol-2-yl)-2-{[(2*S*)-2-hydroxy-3,3-diméthylbutanoyl]amino}pentanamide

**32.** (2*S*)-*N*-(5-{2-[(2,4-diméthylphénoxy)méthyl]phényl}-1,3-thiazol-2-yl)-2-{[(2*S*)-2-hydroxy-3,3-diméthylbutanoyl] amino}pentanamide

**33.** (2*S*)-*N*-(5-{2-[(2,5-diméthylphénoxy)méthyl]phényl}-1,3-thiazol-2-yl)-2-{[(2*S*)-2-hydroxy-3,3-diméthylbutanoyl] amino}pentanamide

**34**. (2*S*)-2-{[(2*S*)-2-hydroxy-3,3-diméthylbutanoyl]amino}-*N*-{4-méthyl-5-[2-(phénoxyméthyl)phényl]-1,3-thiazol-2-yl}pentanamide

**35.** (2*R*)-2-{[(2*S*)-2-hydroxy-3,3-diméthylbutanoyl]amino}-*N*-{5-[2-(phénoxy-méthyl)phényl]-1,3-thiazol-2-yl}pentanamide

**36.** (2*S*)-2-{[(2*S*)-2-hydroxy-3,3-diméthylbutanoyl]amino}-*N*-(5-{2-[(2-méthyl-phénoxy)méthyl]phényl}-1,3-thiazol-2-yl}pentanamide

**37.** (2*S*)-2-{[(2*S*)-2-hydroxy-3-méthylbutanoyl]amino}-*N*-{5-[2-(phénoxyméthyl)-phényl]-1,3-thiazol-2-yl}pentanamide

**38.** (2*S*)-2-[(2-hydroxy-3,3-diméthylbutanoyl)amino]-*N*-(5-{2-[(2-méthoxy-phénoxy)méthyl]phényl}-1,3-thiazol-2-yl) pentanamide

**39.** (2*S*)-*N*-(5-{2-[(2-éthoxyphénoxy)méthyl]phényl}-1,3-thiazol-2-yl)-2-[(2-hydroxy-3,3-diméthylbutanoyl)amino] pentanamide

**40.** (2*S*)-2-[(2-hydroxy-3-phénylpropanoyl)amino]-*N*-{5-[2-(phénoxyméthyl)-phényl]-1,3-thiazol-2-yl}pentanamide

**41.** (2*S*)-*N*-(5-{2-[(2,6-dichlorophénoxy)méthyl]phényl}-1,3-thiazol-2-yl)-2-[(2-hydroxy-3,3-diméthylbutanoyl)amino] pentanamide

**42.** (2*R*)-3-éthyl-2-hydroxy-*N*-{(1*S*)-1-[({5-[2-(phénoxyméthyl)phényl]-1,3-thiazol-2-yl}amino)carbonyl]butyl}pentanamide

**43.** (2*S*)-3-éthyl-2-hydroxy-*N*-{(1*S*)-1-[({5-[2-(phénoxyméthyl)phényl]-1,3-thiazol-2-yl}amino)carbonyl]butyl}pentànamide

**44.** (2*S*)-2-{[(2*S*)-2-hydroxy-3,3-diméthylbutanoyl]amino}-*N*-{5-[2-(isopropoxy-méthyl)-4-méthoxyphényl]-1,3-thiazol-2-yl}pentanamide

**45.** (2*S*)-*N*-(5-{2-[(2-(chloro-6-méthylphénoxy)méthyl]phényl}-1,3-thiazol-2-yl)-2-{[(2*S*)-2-hydroxy-3,3-diméthylbutanoyl]amino}pentanamide

**46.** (2*S*)-*N*-(5-{2-[(2,6-difluorophénoxy)méthyl]phényl}-1,3-thiazol-2-yl)-2-{[(2*S*)2-hydroxy-3,3-diméthylbutanoyl] amino}pentanamide

**47.** (2*S*)-*N*-{5-[4-chloro-2-(phénoxyméthyl)phényl]-1,3-thiazol-2-yl}-2-{[(2*S*)-2-hydroxy-3,3-diméthylbutanoyl]amino}pentanamide

**48.** (2*S*)-*N*-{5-[4-fluoro-2-(phénoxyméthyl)phényl]-1,3-thiazol-2-yl}-2-{[(2*S*)-2-hydroxy-3,3-diméthylbutanoyl]amino}pentanamide

**49.** (2*S*)-2-{[(2*S*)-2-hydroxy-3-méthylbutanoyl]amino}-*N*-{5-[4-méthoxy-2-(phénoxyméthyl)phényl]-1,3-thiazol-2-yl} pentanamide

**50.** (2*S*)-*N*-(5-{2-[(3,4-dichlorophénoxy)méthyl]phényl}-1,3-thiazol-2-yl)-2-{[(2*S*)-2-hydroxy-3,3-diméthylbutanoyl] amino}pentanamide

**51.** (2*S*)-*N*-((1*S*)-1-{[(5-{2-[(cyclohexyloxy)méthyl]phényl}-1,3-thiazol-2-yl)-amino]carbonyl}butyl)-2-hydroxy-4-méthylpentanamide

**52.** (2*S*)-*N*-{5-[4-éthoxy-2-(phénoxyméthyl)phényl]-1,3-thiazol-2-yl}-2-{[(2S)-2-hydroxy-3,3-diméthylbutanoyl]amino}pentanamide

**53.** (2*S*)-*N*-{5-[4-éthoxy-2-(phénoxyméthyl)phényl]-1,3-thiazol-2-yl}-2-{[(2S)-2-hydroxy-3-méthylbutanoyl]amino} pentanamide

**54.** (2*S*)-*N*-{5-[5-fluoro-2-(phénoxyméthyl)phényl]-1,3-thiazol-2-yl}-2-{[(2S)-2-hydroxy-3,3-diméthylbutanoyl]amino}pentanamide

**55.** (2*S*)-*N*-{5-[5-chloro-2-(phénoxyméthyl)phényl]-1,3-thiazol-2-yl}-2-{[(2S)-2-hydroxy-3,3-diméthylbutanoyl]amino}pentanamide

**56.** (2*S*)-*N*-{5-[5-fluoro-2-(phénoxyméthyl)phényl]-1,3-thiazol-2-yl}-2-{[(2S)-2-hydroxy-3-méthylbutanoyl]amino} pentanamide

**57.** (2*S*)-*N*-(5-{2-[(cyclohexyloxy)méthyl]phényl}-1,3-thiazol-2-yl)-2-{[(2S)-2-hydroxy-3,3-diméthylbutanoyl]amino} pentanamide

**58.** (2*S*)-2-hydroxy-3,3-diméthyl-*N*-[(1S)-1-méthyl-2-oxo-2-({5-[2-(phénoxy-méthyl)phényl]-1,3-thiazol-2-yl}amino) éthyl]butanamide

**59.** (2*S*)-2-{[(2S)-2-hydroxy-3,3-diméthylbutanoyl]amino}-*N*-{5-[4-méthyl-2-(phénoxyméthyl)phényl]-1,3-thiazol-2-yl}pentanamide

**60.** (2*S*)-2-{[(2S)-2-hydroxy-3-méthylbutanoyl]amino}-*N*-{5-[5-méthyl-2-(phénoxy-méthyl)phényl]-1,3-thiazol-2-yl} pentanamide

**61.** (2*S*)-*N*-(5-{2-[(3-cyanophénoxy)méthyl]phényl}-1,3-thiazol-2-yl)-2-{[(2S)-2-hydroxy-3,3-diméthylbutanoyl]amino}pentanamide

**62.** (2*S*)-*N*-(5-{2-[(3-fluorophénoxy)méthyl]phényl}-1,3-thiazol-2-yl)-2-{[(2S)-2-hydroxy-3,3-diméthylbutanoyl]amino}pentanamide

**63.** (2*S*)-*N*-(5-{2-[(3-fluorophénoxy)méthyl]phényl}-1,3-thiazol-2-yl)-2-{[(2S)-2-hydroxy-3-méthylbutanoyl]amino} pentanamide

**64.** (2*S*)-2-{[(2S)-2-hydroxy-3,3-diméthy)butanoyl]amino}-*N*-{5-[5-méthyl-2-(phénoxyméthyl)phényl]-1,3-thiazol-2-yl}pentanamide

**65.** (2*S*)-2-{[(2S)-2-hydroxy-3-méthylbutanoyl]amino}-*N*-{5-[4-méthyl-2-(phénoxy-méthyl)phényl]-1,3-thiazol-2-yl} pentanamide

**66.** (2*S*)-*N*-{(1S)-2-[(5-{2-[(cyclohexyloxy)méthyl]phényl}-1,3-thiazol-2-yl)amino]-1-méthyl-2-oxoéthyl}-2-hydroxy-3,3-diméthylbutanamide

**67.** (2*S*)-*N*-(5-{2-[(2-chloro-5-méthylphénoxy)méthyl]phényl}-1,3-thiazol-2-yl)-2-{[(2S)-2-hydroxy-3,3-diméthylbutanoyl]amino}pentanamide

**68.** (2*S*)-2-{[(2S)-2-hydroxy-3,3-diméthylbutanoyl]amino}-*N*-(5-{2-[(3-méthyl-phénoxy)méthyl)phényl}-1,3-thiazol-2-yl)pentanamide

**69.** (2*S*)-*N*-(5-{2-[(2-cyanophénoxy)méthyl]phényl}-1,3-thiazol-2-yl)-2-{[(2S)-2-hydroxy-3,3-diméthylbutanoyl]amino}pentanamide

**70.** (2*S*)-2-{[(2S)-2-hydroxy-3,3-diméthylbutanoyl]amino}-*N*-(5-{2-[(4-pyridinyl-oxy)méthyl]phényl}-1,3-thiazol-2-yl) pentanamide

**71.** (2*S*)-*N*-(5-{2-[(2-chloro-4,5-diméthylphénoxy))méthyl]phényl}-1,3-thiazol-2-yl)-2-{[(2S)-2-hydroxy-3,3-diméthylbutanoyl]amino}pentanamide

**72.** (2*S*)-*N*-(5-{2-[(4-chloro-3-méthylphénoxy))méthyl]phényl}-1,3-thiazol-2-yl)-2-{[(2S)-2-hydroxy-3,3-diméthylbutanoyl]amino}pentanamide

**73.** (2*S*)-*N*-(5-{2-[(2,3-dichlorophénoxy)méthyl]phényl}-1,3-thiazol-2-yl)-2-{[(2S)-2-hydroxy-3,3-diméthylbutanoyl] amino}pentanamide

**74.** (2*S*)-*N*-(5-{2-[(2,3-difluorophénoxy)méthyl]phényl}-1,3-thiazol-2-yl)-2-{[(2S)-2-hydroxy-3,3-diméthylbutanoyl] amino}pentanamide

**75.** (2*S*)-2-{[(2S)-2-hydroxy-3,3-diméthylbutanoyl]amino}-*N*-{4-(4-méthylpentyl)-5-[2-(phénoxyméthyl)phényl]-1,3-thiazol-2-yl)pentanamide

L'invention a également pour objet, parmi les composés de formule générale (I), des composés répondant à la formule générale (I') :

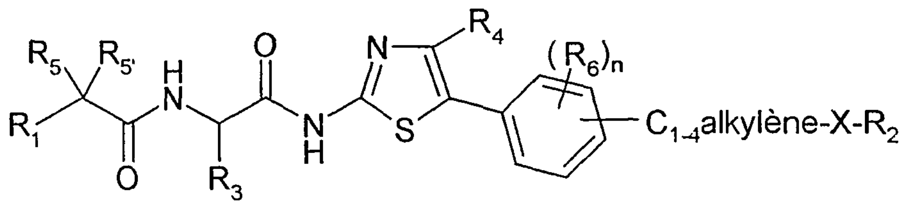

(I')

dans laquelle,

X représente un atome d'oxygène ou de soufre ;

$R_1$ représente un groupe $C_{1-10}$ alkyle éventuellement substitué par un $C_{3-7}$ cycloalkyle, un phényle, un thiényle ; ou $R_1$ représente un groupe $6_{3-7}$ cycloalkyle, thiényle, pyridinyle, pyrimidinyle ;

les groupes thiényle étant éventuellement substitués par un à 3 groupes $C_{1-3}$ alkyle ; le groupe phényle étant éventuellement substitué par un à 5 atomes d'halogène ou groupes $C_{1-3}$ alkyle, $C_{1-3}$ alcoxy, $C_{1-3}$ fluoroalkyle, $C_{1-3}$ fluoroalcoxy ;

$R_2$ représente un groupe $C_{1-6}$ alkyle éventuellement substitué par un groupe $C_{3-7}$ cycloalkyle, phényle, $C_{1-3}$ alcoxy, hydroxy ; ou $R_2$ représente un groupe $C_{3-7}$ cycloalkyle, pipéridinyle, phényle ou pyridinyle ;

les groupes $C_{3-7}$ cycloalkyle et pipéridinyle étant éventuellement substitués par un ou plusieurs groupes $C_{1-3}$ alkyle, $C_{1-3}$ alcoxy, hydroxy, $C_{1-3}$ fluoroalkyle, $C_{1-3}$ fluoroalcoxy ;

les groupes phényle et pyridinyle étant éventuellement substitués par un ou plusieurs atomes d'halogène ou groupes $C_{1-3}$ alkyle, $C_{1-3}$ alcoxy, hydroxy, $C_{1-3}$ fluoroalkyle, $C_{1-3}$ fluoroalcoxy ;

$R_3$ représente un atome d'hydrogène ou un groupe $C_{1-6}$ alkyle éventuellement substitué par un groupe $C_{3-7}$ cycloalkyle ;

$R_4$ représente un atome d'hydrogène ou un groupe $C_{1-4}$ alkyle ;

$R_5$ et $R_{5'}$ représentent, indépendamment l'un de l'autre, un atome d'hydrogène, d'halogène, un groupe hydroxy, $C_{1-3}$ alkyle; ou $R_5$ et $R_{5'}$ forment ensemble un groupe oxo ou oxime tel que :

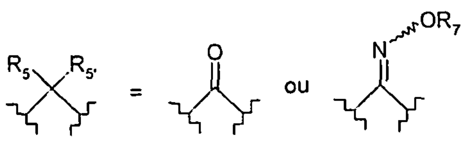

où $R_7$ représente un atome d'hydrogène ou un $C_{1-3}$ alkyle ;

n représente un nombre entier allant de 0 à 3 ; et

$R_6$ représente, indépendamment l'un de l'autre lorsque n = 2 ou 3, un atome d'hydrogène, d'halogène, un groupe hydroxy, $C_{1-3}$ alkyle, $C_{1-3}$ alcoxy, $C_{1-3}$ fluoroalkyle ou $C_{1-3}$ fluoroalcoxy.

Parmi les composés de formule générale (I'), un sous-groupe de composés préférés est constitué par les composés pour lesquels :

- X représente un atome d'oxygène ou de soufre; et/ou
- $R_1$ représente un groupe $C_{1-5}$ alkyle de préférence un méthyle, éthyle, 1-méthyléthyle, 1,1-diméthyléthyle, propyle, 1-méthylpropyle, 2-méthylpropyle, 1-éthylpropyle, éventuellement substitué par un phényle, un thiényle ; ou $R_1$ représente un groupe $C_{3-7}$ cycloalkyle, de préférence un cyclohexyle, un groupe thiényle, pyridinyle ; les groupes thiényle étant éventuellement substitués par un ou deux groupes $C_{1-3}$ alkyle, de préférence méthyle ; le groupe phényle étant éventuellement substitué par un ou deux atomes d'halogène, de préférence chlore ou fluor ; et/ou
- $R_2$ représente un groupe $C_{1-6}$ alkyle, de préférence un éthyle, 1-méthyléthyle ; ou $R_2$ représente un groupe $C_{3-7}$ cycloalkyle, de préférence un cyclohexyle, ou phényle ;
  le groupe phényle étant éventuellement substitué par un ou deux groupes $C_{1-3}$ alkyle, de préférence méthyle, éthyle, $C_{1-3}$ alcoxy, de préférence méthoxy, éthoxy, hydroxy, fluoroalcoxy, de préférence trifluorométhoxy, ou par un ou deux atomes d'halogène, de préférence chlore, fluor ; et/ou
- $R_3$ représente un groupe $C_{1-6}$ alkyle, de préférence un éthyle, propyle; et/ou
- $R_4$ représente un atome d'hydrogène ou un groupe $C_{1-4}$ alkyle, de préférence un méthyle ; et/ou
- $R_5$ et $R_{5'}$ représentent, indépendamment l'un de l'autre, un atome d'hydrogène, un hydroxy; ou $R_5$ et $R_{5'}$ forment ensemble un groupe oxo ; et/ou

- $R_6$ représente un atome d'hydrogène, d'halogène, de préférence un chlore ou un fluor, un $C_{1-3}$alcoxy, de préférence un méthoxy ; et/ou
- n est égal à 0 ou 1.

Dans le cadre de l'invention, on entend par :

- $C_{t-z}$ où t et z peuvent prendre les valeurs de 1 à 10, une chaîne carbonée pouvant avoir de t à z atomes de carbone, par exemple $C_{1-3}$ une chaîne carbonée qui peut avoir de 1 à 3 atomes de carbone, $C_{3-6}$ une chaîne carbonée qui peut avoir de 3 à 6 atomes de carbone,... ;
- alkyle, un groupe aliphatique saturé, linéaire ou ramifié, par exemple un groupe $C_{1-6}$ alkyle représente une chaîne carbonée de 1 à 6 atomes de carbone, linéaire ou ramifiée, plus particulièrement un méthyle, éthyle, propyle, 1-méthyléthyle, butyle, 1-méthylpropyle, 2-méthylpropyle, 1,1-diméthyléthyle,..... de préférence un méthyle, éthyle, propyle ou 1-méthyléthyle ;
- alkylène, un groupe alkyle divalent ;
- cycloalkyle, un groupe alkyle cyclique, par exemple, un groupe $C_{3-7}$ cycloalkyle représente un cycle carboné de 3 à 7 atomes de carbone, plus particulièrement un cyclopropyle, cyclobutyle, cyclopentyle, cyclohexyle, cycloheptyle, de préférence un cyclopentyle ou cyclohexyle ;
- alcoxy, un groupe -O-alkyle où le groupe alkyle est tel que précédemment défini ;
- fluoroalkyle, un groupe alkyle dont un ou plusieurs atomes d'hydrogène ont été substitués par un atome de fluor ;
- fluoroalcoxy, un groupe alcoxy dont un ou plusieurs atomes d'hydrogène ont été substitués par un atome de fluor ; et
- atome d'halogène, un fluor, un chlore, un brome ou un iode.

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans ce qui suit, on entend par groupe partant, un groupe pouvant être facilement clivé d'une molécule, avec départ d'une paire électronique, par rupture d'une liaison hétérolytique. Ce groupe peut ainsi être remplacé facilement par un autre groupe lors d'une réaction de substitution par exemple. De tels groupes partants sont, par exemple, les halogènes, ou un groupe hydroxy activé tel qu'un mésylate, tosylate, triflate, acétyle, ... etc. Des exemples de groupes partants ainsi que des références pour leur préparation sont donnés dans « Advanced Organic Chemistry », J. March, 3rd Edition, Wiley Interscience, p 310-316.

On entend par groupe protecteur, un groupe permettant d'empêcher la réactivité d'une fonction ou position, lors d'une réaction chimique pouvant l'affecter, et qui restitue la molécule après clivage selon des méthodes connues de l'homme du métier. Des exemples de groupes protecteurs ainsi que les méthodes de protection et déprotection sont données, entre autres, dans Protective groups in Organic Synthesis, Greene et al., 2nd Ed. (John Wiley & Sons, Inc., New York).

L'invention a pour second objet des procédés de préparation des composés de formule (I).

Ainsi, ces composés peuvent être préparés par les procédés, illustrés dans les schémas qui suivent, dont les conditions opératoires sont classiques pour l'homme du métier.

## Schéma 1

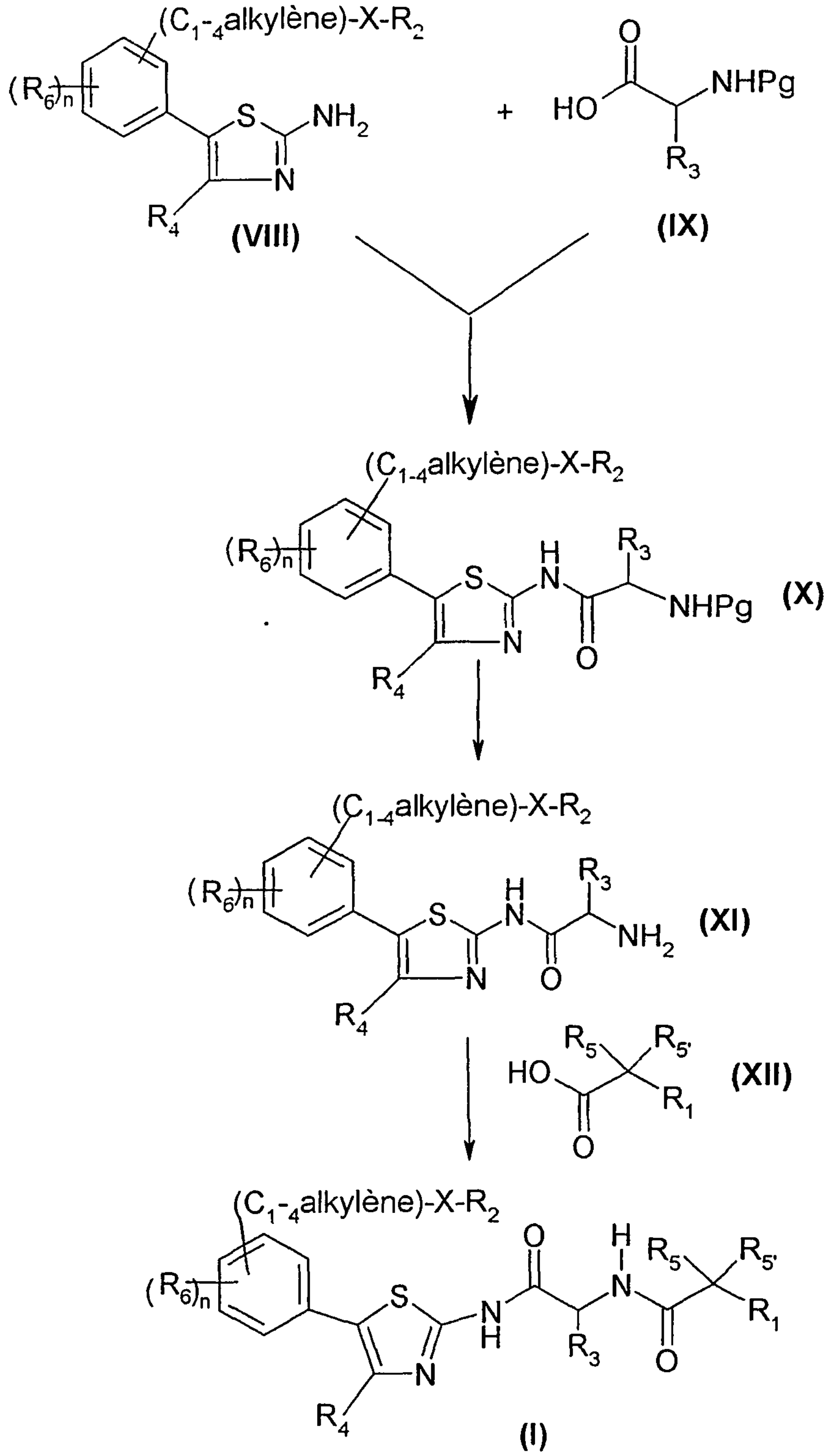

(VIII) (IX) (X) (XI) (XII) (I)

Selon le schéma 1, le composé de formule (I) peut être obtenu par couplage peptidique de l'amine de formule (XI) avec l'acide de formule (XII) selon des conditions connues de l'homme du métier, par exemple en présence d'hexafluorophosphate de benzotriazol-1-yloxy-tris(diméthylamino)phosphonium (BOP) et de *N*-éthylmorpholine ou *N*-méthylmorpholine dans un solvant inerte tel que le diméthylformamide, l'acétonitrile ou le dichlorométhane à une température pouvant aller de 0°C à la température ambiante.

L'amine de formule (XI) est obtenue par couplage peptidique de l'amine de formule (VIII) avec l'aminoacide de formule (IX), dans laquelle Pg représente un groupe protecteur, dans les conditions telles que décrites ci-dessus, pour donner le composé de formule (X). L'aminoacide de formule (IX) est, par exemple, protégé au moyen d'un *N-tert*-butyloxycarbonyl (Boc). Le composé (X) est ensuite déprotégé selon des méthodes connues de l'homme du métier, pour donner l'amine de formule (XI). Par exemple, si le groupe protecteur utilisé est le Boc, celui-ci peut être déprotégé par hydrolyse acide, en présence d'acide chlorhydrique gazeux anhydre.

Le composé de formule (VIII) peut être préparé selon le schéma 2.

## Schéma 2

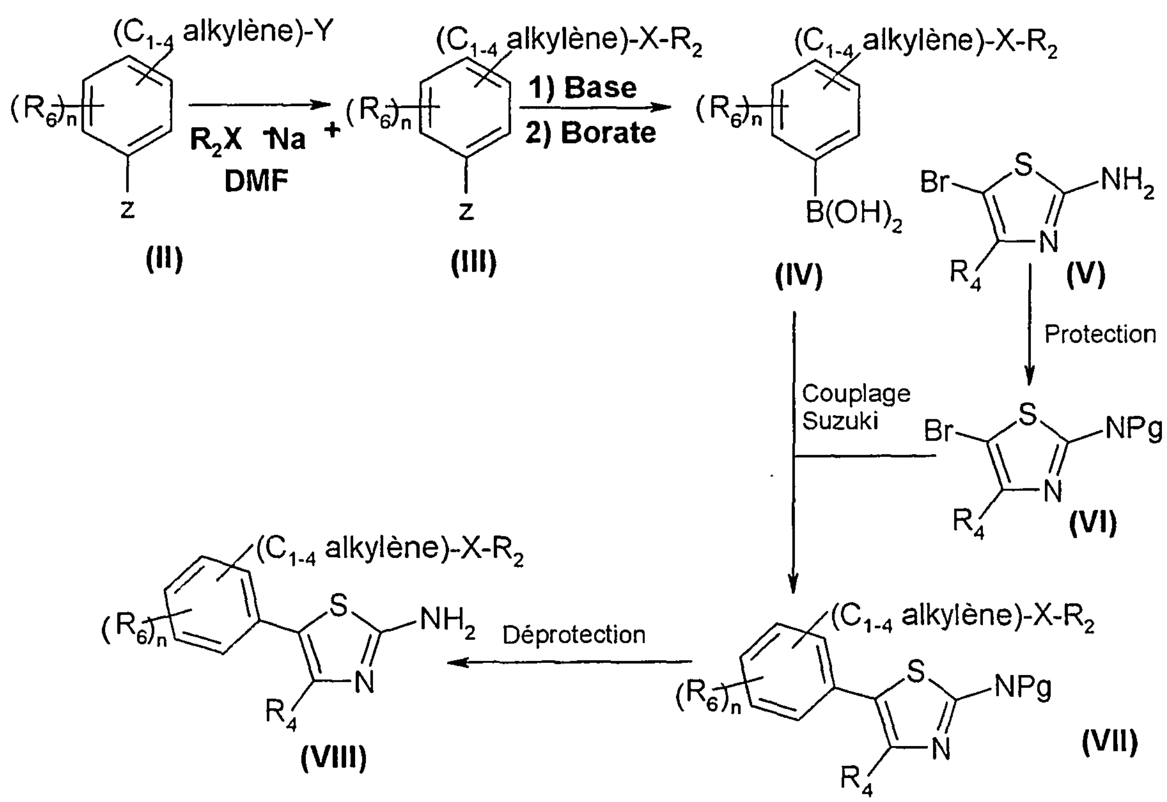


Selon ce schéma, l'aralkyle de formule (II), dans laquelle Y représente un groupe partant, de préférence un atome d'halogène tel que le brome et Z représente un atome d'halogène tel que le brome, est condensé avec un alcoolate ou un thiolate alcalin, par exemple, de formule $R_2X^-Na^{\oplus}$ dans laquelle X représente un atome d'oxygène ou de soufre. La réaction est réalisée dans un solvant inerte tel que le diméthylformamide à une température pouvant aller de 0°C à 50°C, pour conduire au composé de formule (III). L'aryle de formule (III) est transformé en acide boronique de formule (IV) selon une adaptation du procédé décrit par Schoevaars, J. Am. Chem. Soc., 1999, 121, 9550-9561. La transformation peut par exemple être réalisée par formation préalable de l'anion du composé de formule (III), par exemple par action d'une base forte telle que le butyllithium, dans un solvant éthéré tel que le tétrahydrofurane, à des températures pouvant aller de -50°C à -80°C. Cet anion est ensuite mis en réaction avec un borate tel que le triméthylborate pour donner après hydrolyse, l'acide boronique de formule (IV).

Le couplage de l'acide boronique (IV) avec le 5-bromo-thiazole de formule (VI) dans laquelle Pg représente un groupe protecteur, tel qu'un imino par exemple une diphénylcétone imine, peut être effectué selon la réaction de Suzuki, par une adaptation du procédé décrit par Wolfe, J. Org. Chem., 1997, 62, 4943-4948, pour conduire au 5-phényl-thiazole de formule (VII). Le couplage est réalisé par exemple dans un solvant éthéré tel que le dioxane en présence de phosphate de tripotassium trihydrate et d'un catalyseur tel que le tétrakis(triphénylphosphine) de palladium (0) à une température pouvant aller de la température ambiante à la température de reflux du solvant. Le 5-phényl-thiazole de formule (VII)

ainsi préparé est ensuite déprotégé selon des méthodes connues de l'homme du métier pour donner le 5-phényl-2-amino-thiazole de formule (VIII).

Le 5-bromo-thiazole de formule (VI) est obtenu par protection de la fonction amino du composé de formule (V) correspondant. De préférence, elle est protégée sous forme de diphénylcétone imine dans des conditions connues de l'homme du métier.

Les composés de départ, notamment les composés de formule (II), (V), (IX) et (XII), quand leur mode de préparation n'est pas décrit, sont disponibles dans le commerce ou décrits dans la littérature, ou peuvent être préparés par des méthodes qui y sont décrites ou qui sont connues de l'homme du métier.

Par exemple, le 5-bromo-2-amino-thiazole (V), peut être obtenu par bromation du 2-amino-thiazole correspondant selon une adaptation du procédé décrit par Kaye, J. Chem. Soc. Perkins I, 1981, 2335-2339.

Par exemple, le composé de formule (XII) peut être obtenu par adaptation des procédés décrits par Middleton et al., J. Org. Chem., 45, 14, 1980, 2883-2887 et par Miyamoto et al., J. Amer. Chem. Soc., 114, 15, 1992, 6256-6257.

Les significations de X, $R_1$, $R_2$, $R_3$, $R_4$, $R_5$, $R_{5'}$, $R_6$ et n dans les composés de formule (II) à (XII) sont telles que définies pour les composés de formule (I).

Les exemples suivants décrivent la préparation de certains composés conformes à l'invention. Ces exemples ne sont pas limitatifs et ne font qu'illustrer l'invention. Les numéros des composés exemplifiés renvoient à ceux donnés dans le tableau ci-après. Les micro-analyses élémentaires et les spectres RMN, IR ou de masse confirment les structures des composés obtenus.

**Exemple 1 (composé n°9)**

**(2S)-2-{[(2S)-2-hydroxy-3,3-diméthylbutanoyl]amino}-N-{5-[2-(phénoxy-méthyl)phényl]-1,3-thiazol-2-yl}pentanamide**

**1.1. 1-bromo-2-(phénoxyméthyl)benzène**

A 20,2 g de phénol en solution dans 150 ml de diméthylformamide, on additionne à 5°C par portions 1,2 g d'hydrure de sodium (à 50 % en suspension dans l'huile). On agite à température ambiante et on introduit, à 5°C 37,2 g de bromure de 2-bromobenzyle en solution dans 15 ml de diméthylformamide. Après 2 heures à 20°C, on verse le milieu réactionnel sur de l'eau glacée et on extrait à l'acétate d'éthyle. On sèche la phase organique sur sulfate de sodium anhydre et on concentre pour obtenir 36 g d'huile.

RMN$^1$H $\delta$ en ppm (DMSO $d_6$) : 5,22 (s, 2H) ; 7,09 - 7,67 (massif, 9H).

**1.2 Acide 2 -(phénoxyméthyl)phénylboronique**

A 36 g de 1-bromo-2-(phénoxyméthyl)benzène, obtenu à l'étape 1.1, en solution dans 150 ml de tétrahydrofurane, on additionne goutte à goutte à -70°C, 90 ml de n-butyllithium (1,6 M) en solution dans l'hexane. Après 2 heures à -70°C, on introduit goutte à goutte 16 ml de triméthylborate. On laisse remonter la température du milieu réactionnel à -30°C. On hydrolyse avec une solution saturée en chlorure d'ammonium, puis on extrait à l'acétate d'éthyle et on sèche la phase organique avec du sulfate de sodium anhydre. Après évaporation, on obtient 33 g de solide blanc.

RMN$^1$H $\delta$ en ppm (DMSO $d_6$) : 5,25 (s, 2H) ; 6,85 - 7,67 (massif, 11 H).

**1.3 5-bromo-N-(diphénylméthylène)-1,3-thiazol-2-amine**

A 34 g de 5-bromo-1,3-thiazol-2-aminebromohydrate, en suspension dans 300 ml de 1,2-dichloroéthane, on additionne 26 g de benzophénone imine. On maintient pendant 18 heures à reflux. On filtre le précipité formé et on concentre le filtrat pour obtenir 37,2 g de solide.

PF = 109°C

RMN$^1$H $\delta$ en ppm (DMSO $d_6$): 7,34 (m, 2H) ; 7,50 - 7,76 (massif, 9H).

**1.4 5-{2-[(phénoxy)méthyl]phényl}-N-(diphénylméthylène)-1,3-thiazol-2-amine**

A 14,8 g d'acide 2-(phénoxyméthyl)phénylboronique, obtenu à l'étape 1.2, en solution dans 250 ml de 1,4-dioxane, on introduit successivement 15 g de phosphate de tripotassium dihydrate, 10,5 g de 5-bromo-N-(diphénylméthylène)-1,3-thiazol-2-amine obtenu à l'étape 1.3, 1,5 g de tétrakis(triphénylphosphine) de palladium (0) et on maintient 1 heure à reflux. On évapore le milieu réactionnel à sec, on le reprend à l'acétate d'éthyle et on le lave à l'eau. On sèche la phase organique sur sulfate de sodium anhydre et on concentre les solvants. On chromatographie le résidu sur une colonne de gel de silice en éluant au dichlorométhane pour obtenir 35 g d'huile jaune.

RMN$^1$H δ en ppm : 4,81 (s, 2H) ; 7,17 - 7,83 (massif, 20H)

### 1.5 5-{2-[(phénoxy)méthyl]phényl}-1,3-thiazol-2-amine

A 35 g de 5-{2-[(phénoxy)méthyl]phényl}-N-(diphénylméthylène)-1,3-thiazol-2-amine, obtenu à l'étape 1.4, en solution dans 250 ml de méthanol, on additionne 150 ml d'une solution aqueuse d'acide chlorhydrique (1M) et on agite 18 heures à 20°C. On évapore à sec, on reprend le résidu à l'éther diéthylique et on lave avec une solution aqueuse d'hydroxyde de sodium (0,5 M). On sèche la phase organique sur sulfate de sodium anhydre et on concentre. Le résidu est chromatographié sur une colonne de gel de silice en éluant avec un mélange dichlorométhane/méthanol 98/2 (v/v), pour obtenir 15 g d'une solide beige.
PF = 154°C
RMN$^1$H δ en ppm (DMSO $d_6$) : 5,07 (s, 2H) ; 6,98 - 7,65 (massif, 10H).

### 1.6 *tert*-butyl-(1S)-1-[({5-[2-(phénoxyméthyl)phényl]-1,3-thiazol-2-yl}-amino)carbonyl]butylcarbamate

A 3,35 g d'acide (2*S*)-2-[(*tert*-butyloxycarbonyl)amino]pentanoïque en solution dans 35 ml de diméthylformamide, on additionne à 0°C, 7,1 g d'hexafluorophosphate de benzotriazol-1-yloxy-tris(diméthylamino)phosphonium puis, goutte à goutte, 2,1 ml de N-méthylmorpholine. Après 15 minutes à 0°C, on introduit 4 g de 5-{2-[(phénoxy)méthyl] phényl}-1,3-thiazol-2-amine, obtenu à l'étape 1.5, et on agite 18 heures à température ambiante. On reprend le milieu à l'acétate d'éthyle et on lave 2 fois à l'eau. On sèche la phase organique sur sulfate de sodium anhydre et on concentre. On chromatographie le résidu sur une colonne de gel de silice en éluant avec un mélange dichlorométhane/méthanol 98/2 (v/v) pour obtenir 5,2 g d'huile incolore.
RMN$^1$H δ en ppm (DMSO $d_6$) : 0,88 (t, 3H) ; 1,22 - 1,65 (massif, 13H) ; 4,24 (q, 1 H) ; 5,09 (s, 2H) ; 6,94 - 7,67 (massif, 10H) ; 12,23 (s, 1 H).

### 1.7 chlorhydrate de (2S)-2-amino-N-(5-{2-[(phénoxy)méthyl]phényl}-1,3-thiazol-2-yl)pentanamide

A 5 g de *tert*-butyl-(1S)-2-[(5-{2-[(phénoxy)méthyl]phényl}-1,3-thiazol-2-yl)-amino]-1-propyl-2-oxoéthylcarbamate, obtenu à l'étape 1.6, en solution dans 60 ml d'acétate d'éthyle, on additionne goutte à goutte à 0°C, 25 ml d'une solution d'acide chlorhydrique gaz (4,5 M) dans l'acétate d'éthyle. On agite 18 heures à 20°C. On filtre le précipité formé, on le rince 2 fois à l'éther diéthylique et on le sèche pour obtenir 3 g d'un solide blanc.
PF = 148°C
RMN$^1$H δ en ppm (DMSO $d_6$) : 0,90 (t, 3H) ; 1,39 (m, 2H) ; 1,85 (m, 2H) ; 4,18 (q, 1 H) ; 5,08 (s, 2H) ; 6,94 - 7,68 (massif, 10H) ; 8,65 (s, 3H).

### 1.8 (2S)-2-{[(2S)-2-hydroxy-3,3-diméthylbutanoyl]amino}-N-{5-[2-(phénoxyméthyl)phényl]-1,3-thiazol-2-yl} pentanamide

A 0,32 g d'acide (2*S*)-2-hydroxy-3,3-diméthylbutanoïque en solution dans 25 ml de diméthylformamide à 0°C on additionne successivement 1,36 g d'hexo-fluorophosphate de benzotriazol-1-yloxytripyrrolidine phosphonium et 0,7 ml de N-éthylmorpholine. Après 20 minutes à 0°C, on introduit 0,88 g de chlorhydrate de (2*S*)-2-amino-N-(5-{2-[(phénoxy) méthyl]phényl}-1,3-thiazol-2-yl)pentanamide, obtenu à l'étape 1.7, et on agite pendant 18 heures à température ambiante. Le milieu réactionnel est repris à l'acétate d'éthyle et lavé à l'eau. La phase organique est séchée sur sulfate de sodium anhydre et concentrée. Le résidu est chromatographié sur une colonne de gel de silice en éluant avec un mélange dichlorométhane/ méthanol 99/1 (v/v) pour obtenir, après cristallisation à l'éther isopropylique, 0,83 g de solide blanc.
PF = 84°C
RMN$^1$H δ en ppm (DMSO $d_6$) : 0,89 (t, 3H) ; 0,93 (s, 9H) ; 1,33 (m, 2H) ; 1,71 (q, 21 H) ; 3,57 (d, 1 H) ; 4,61 (q, 1 H) ; 5,09 (s, 2H) ; 5,61 (d, 1 H) ; 6,97-7,02 (massif, 3H) ; 7,28-7,67 (massif, 7H) ; 7,81 (d, 1 H) ; 12,28 (s, 1 H).

$[\alpha]_D^{20} = -81{,}8$ ($c = 1/CH_3OH$).

**Exemple 2 (composé n°16)**

**(2*S*)-2-{[2-(2,5-diméthyl-3-thiényl)acétyl]amino}-*N*-{5-[2-(éthoxyméthyl)-phényl]-1,3-thiazol-2-yl}pentanamide**

2.1 2-(2,5-diméthyl-3-thiényl)-1-(4-morpholinyl)-1-éthanethione

A 5 g de 2,5-diméthyl-3-acétylthiophène, on additionne 1,68 g de soufre, 6,5 ml de morpholine et on porte 10 heures à reflux. On ramène à 20°C et on verse sur une solution aqueuse d'acide chlorhydrique (1 N). On extrait le milieu à l'acétate d'éthyle. On sèche la phase organique sur sulfate de sodium anhydre et on la concentre. On purifie le résidu par chromatographie sur colonne de gel de silice en éluant avec un mélange cyclohexane/acétate d'éthyle 8/2 (v/v) pour obtenir 6,8 g d'huile orangée.
RMN$^1$H $\delta$ en ppm (DMSO $d_6$) : 2,30 (s, 3H) ; 2,34 (s, 3H) ; 3,47 (t, 2H) ; 3,65 (m, 4H) ; 4,07 (s, 2H) ; 4,20 (t, 2H) ; 6,56 (s, 1 H).

**2.2 acide 2-(2,5-diméthyl-3-thiényl)acétique**

L'acide 2-(2,5-diméthyl-3-thiényl)acétique est préparé selon une méthode décrite dans Heterocycl. Chem ; EN ; 25 ; 1988 ; 1571-1581.
A 6,7 g de 2-(2,5-dimethyl-3-thiényl)-1-(4-morpholinyl)-1-éthanethione, obtenu à l'étape 2.1, en solution dans 70 ml de méthanol, on additionne 21 ml d'une solution aqueuse d'hydroxyde de sodium (50 % massique) et on porte 6 heures à reflux. Après évaporation du méthanol, on dilue le résidu à l'eau et on acidifie avec une solution aqueuse d'acide chlorhydrique (6N). On filtre le précipité formé puis on le chromatographie sur une colonne de gel de silice en éluant au dichlorométhane pour obtenir 3,6 g de cristaux beiges.
PF = 65°C
RMN$^1$H $\delta$ en ppm (DMSO $d_6$) : 2,26 (s, 3H) ; 2,34 (s, 3H) ; 3,39 (s, 2H) ; 6,56 (s, 1 H).

**2.3 (2*S*)-2-{[(2,5-diméthyl-3-thiényl)acétyl]amino}-*N*-{5-[2-(éthoxyméthyl)-phényl]-1,3-thiazol-2-yl}pentanamide**

On procède de la même manière que dans l'étape 1.8 de l'exemple 1 en remplaçant l'acide (2*S*)-2-hydroxy-3,3-diméthylbutanoïque par l'acide 2-(2,5-diméthyl-3-thiényl)acétique, obtenu à l'étape 2.2. On obtient 0,67g de cristaux blancs.
PF = 84°C.
RMN$^1$H $\delta$ en ppm (DMSO d 6) :0,89 (t, 3H) ; 1,16 (t, 3H) ; 1,29 (m, 2H) ; 1,65 (m, 2H) ; 2,25 (s, 3H) ; 2,30 (s, 3H) ; 3,33 (m, 2H) ; 3,47 (q, 2H) ; 4,44 (s, 2H) ; 4,49 (q, 1 H) ; 4,55 (s, 1 H) ; 7,35-7,55 (massif, 5H) ; 8,32 (d, 1 H) ; 12,28 (s, 1 H).

$$[\alpha]_D^{20} = -103\ (c = 1/CH_3OH).$$

**Exemple 3 (composé n°22)**

**(2*S*)-2-{[2-(5-méthyl-2-thiényl)acétyl]amino}-*N*-{5-[2-(phénoxyméthyl)phényl]-1,3-thiazol-2-yl}pentanamide**

**3.1 2-(5- méthyl-2-thiényl)-1-(4-morpholinyl)-1-éthanethione**

Le 2-(5- méthyl-2-thiényl)-1-(4-morpholinyl)-1-éthanethione est préparé selon une méthode analogue à celle décrite dans l'exemple 2.1.
RMN$^1$H $\delta$ en ppm (DMSO $d_6$) : 2,40 (s, 3H) ; 3,49 (t, 2H) ; 3,64 (t, 2H) ; 3,81 (t, 2H) ; 4,19 (t, 2H) ; 4,40 (s, 2H) ; 6,49 (d, 1 H) ; 6,77 (d, 1 H).

**3.2 acide 2-(5-méthyl-2-thiényl)acétique**

L'acide 2-(5-méthyl-2-thiényl)acétique est préparé selon une méthode analogue à celle décrite dans l'exemple 2.2.
PF = 54°C
RMN$^1$H $\delta$ en ppm (DMSO $d_6$) : 2,38 (s, 3H) ; 3,72 (s, 3H) ; 6,61 (d, 1 H) ; 6,69 (d, 1H).

**3.3 (2*S*)-2-{[2-(5-méthyl-2-thiényl)acétyl]amino}-*N*-{5-[2-(phénoxy-méthyl)phényl]-1,3-thiazol-2-yl}pentanamide**

On procède de la même manière que dans l'étape 1.8 de l'exemple 1 en remplaçant l'acide (2*S*)-2-hydroxy-3,3-diméthylbutanoïque par l'acide 2-(5-méthyl-2-thiényl)acétique, obtenu à l'étape 3.2. On obtient 0,73g de cristaux beiges.
PF = 81 °C.
RMN$^1$H $\delta$ en ppm (DMSO d 6) :0,87 (t, 3H) ; 1,32 (m, 2H) ; 1,64 (m, 2H) ; 2,36 (s, 3H) ; 3,62 (q, 2H) ; 4,48 (q, 1 H) ; 5,07 (s, 2H) ; 6,59 (d, 1 H) ; 6,67 (d, 1 H) ; 6,95-7 (massif, 3H) ; 7,27-7,73 (massif, 7H) ; 8,41 (d, 1 H) ; 12,30 (s, 1 H).

$$[\alpha]_D^{20} = -91,7 \ (c = 1/CH_3OH).$$

**Exemple 4 (composé n°7)**

**(2*S*)-*N*-{5-[2-(isopropoxyméthyl)phényl]-1,3-thiazol-2-yl}-2-{[3-(3-thiényl)-propanoyl]amino}pentanamide**

**4.1 acide (*E*)-3-(3-thiényl)-2-propènoïque**

A 25 g de 3-thiénaldéhyde en solution dans 100 ml de pyridine, on additionne 46 g d'acide malonique, 2 ml de pipéridine et on porte à 100°C pendant 4 heures. On refroidit le milieu réactionnel à 30°C et on le verse sur une solution aqueuse d'acide chlorhydrique (2N). On filtre le précipité formé et on le rince à l'éther isopropylique pour obtenir après séchage 30 g d'un solide blanc.
PF = 152°C
RMN$^1$H $\delta$ en ppm (DMSO $d_6$) : 6,36 (d, 1H) ; 7,51-7,62 (massif, 3H) ; 7,93 (d, 1 H) ; 12,27 (s, 1 H).

**4.2 (*E*)-3-(3-thiényl)-2- propènoate d'éthyle**

A 11 g d'acide (*E*)-3-(3-thiényl)-2-propènoïque, obtenu à l'étape 4.1, en solution dans 50 ml de diméthylformamide on additionne 11,5 g de carbonate de potassium, 6,8 ml d'iodoéthane et on agite pendant 48 heures à 20°C. On reprend le milieu à l'acétate d'éthyle et on le lave à l'eau. On sèche la phase organique sur sulfate de sodium anhydre et on la concentre pour obtenir 12,5 g d'huile.
RMN$^1$H $\delta$ en ppm (DMSO $d_6$) : 1,26 (t, 3H) ; 4,17 (q, 2H) ; 6,47 (d, 1H) ; 7,57-7,71 (massif, 3H) ; 8,01 (d, 1 H).

**4.3 3-(3-thiényl)-2-propanoate d'éthyle**

A 12,5 g de (*E*)-3-(3-thiényl)-2-propènoate d'éthyle, obtenu à l'étape 4.2, en solution dans 100 ml d'éthanol, on additionne 4 g de palladium sur charbon à 10 % et on agite pendant 24 heures à 60°C sous 5 bars d'hydrogène. On filtre le catalyseur et on concentre le filtrat pour obtenir 11 g d'huile.
RMN$^1$H $\delta$ en ppm (DMSO $d_6$) : 1,20 (t, 3H) ; 2,62 (t, 2H) ; 2,88 (t, 2H) ; 4,07 (q, 2H) ; 7,02 (d, 1 H) ; 7,18 (m, 1 H) ; 7,45 (m, 1 H).

**4.4 acide 3-(3-thiényl)propanoïque**

A 11 g de 3-(3-thiényl)-2-propanoate d'éthyle, obtenu à l'étape 4.3, en solution dans 100 ml d'éthanol, on additionne 75 ml d'une solution aqueuse d'hydroxyde sodium (2N). On agite pendant 18 heures à 20°C. Après évaporation des solvants, le résidu est acidifié. Le précipité formé est filtré et séché sous vide pour obtenir 6,3 g d'un solide beige.
PF = 59°C
RMN$^1$H $\delta$ en ppm (DMSO $d_6$) : 2,56 (t, 2H) ; 2,85 (t, 2H) ; 7,02 (t, 1H) ; 7,18 (s, 1 H) ; 7,45 (m, 1 H) ; 12,14 (s, 1 H).

**4.5 (2*S*)-*N*-{5-[2-isopropoxyméthyl)phényl]-1,3-thiazol-2-yl}-2-{[3-(3-thiényl)propanoyl]amino}pentanamide**

On procède de la même manière que dans l'étape 1.8 de l'exemple 1 en remplaçant l'acide (2*S*)-2-hydroxy-3,3-diméthylbutanoïque par l'acide 3-(3-thiényl)propanoïque, obtenu à l'étape 4.4. On obtient 0,75g de cristaux beiges.
PF = 101 °C.
RMN$^1$H $\delta$ en ppm (DMSO d 6) :0,87 (t, 3H) ; 1,13 (d, 6H) ; 1,33 (m, 2H) ; 1,62 (m, 2H) ; 2,48 (t, 2H) ; 2,82 (t, 2H) ; 3,67 (m, 1 H) ; 4,45 (s, 2H) ; 4,54 (q, 1H) ; 6,98 (d, 1H) ; 7,12 (d, 1 H) ; 7,35-7,57 (massif, 6H) ;8,21 (d, 1 H) ; 12,26 (s, 1 H).

$$[\alpha]_D^{20} = -71 \ (c = 1/CH_3OH).$$

**Exemple 5**

**(2*R*)-3-éthyl-2-hydroxy-*N*-{(1*S*)-1-[({5-[2-(phénoxyméthyl)phényl]-1,3-thiazol-2-yl}amino)carbonyl]butyl}pentanamide (composé n˚42) et (2*S*)-3-éthyl-2-hydroxy-*N*-{(1*S*)-1-[({5-[2-(phénoxyméthyl)phényl]-1,3-thiazol-2-yl} amino)carbonyl]butyl}pentanamide (composé n˚43)**

**5.1 acide 3-éthyl-2-hydroxypentanoïque**

A une solution de 1,24 ml de 2-éthylbutyraldéhyde dans 18 ml de dichlorométhane anhydre, on additionne avec précaution 1,5 ml de cyanure de triméthylsilyle puis une quantité catalytique de iodure de zinc. On agite le milieu réactionnel pendant 2 heures à température ambiante puis à 60˚C durant 3.5 heures. On refroidit le milieu réactionnel à 0˚C et on additionne 3,5 ml d'acide chlorhydrique concentré. On agite le milieu réactionnel pendant 18 heures à température ambiante puis 1 heure à reflux. Après refroidissement, on verse le mélange réactionnel dans de l'eau et on extrait deux fois avec 50 ml d'acétate d'éthyle. On extrait les phases organiques combinées avec 100 ml d'hydroxyde de sodium (7.5N) à 4˚C. Après séparation, on lave la phase aqueuse avec 3 fois 50 ml d'acétate d'éthyle. On acidifie la phase aqueuse avec 70 ml d'acide chlorhydrique (12N) et on extrait avec 3 fois 50 ml d'acétate d'éthyle. On sèche les phases organiques rassemblées et on évapore le solvant. PF = 84˚C

**5.2 (2*R*)-3-éthyl-2-hydroxy-*N*-{(1*S*)-1-[({5-[2-(phénoxyméthyl)phényl]-1,3-thiazol-2-yl}amino)carbonyl]butyl} pentanamide et (2*S*)-3-éthyl-2-hydroxy-*N*-{(1*S*)-1-[({5-[2-(phénoxyméthyl)phényl]-1,3-thiazol-2-yl}amino)carbonyl]-butyl}pentanamide**

On procède de la même manière que dans l'étape 1.8 de l'exemple 1 en remplaçant l'acide (2*S*)-2-hydroxy-3,3-diméthylbutanoïque par l'acide 3-éthyl-2-hydroxypentanoïque, obtenu à l'étape 5.1. On obtient 0,78 g de solide blanc.

**Composé n˚42 (SR) :**

PF = 67,4˚C.
RMN$^1$H δ en ppm (DMSO d 6) :0,74 (t, 3H) ; 0,84-0,90 (massif, 6H) ; 1,26 - 1,71 (massif, 9H); 3,94 (m, 1H); 4,57 (q, 1H) ; 5,07 (s, 2H); 5,40 (s, 1H); 6,94-6,99 (massif, 3H) ; 7,26-7,93 (massif, 7H) ;7,91 (d, 1H); 12,27 (s, 1H).

$$[\alpha]_D^{20} = -41{,}5 \ (c = 1/CH_3OH).$$

**Composé n˚43 (SS) :**

PF = 122,5˚C.
RMN $^{1H}$ δ en ppm (DMSO d 6) : 0,78-0,89 (massif, 9H) ; 1,18-1,38 (massif, 6H) ; 1,55 (m, 1 H) ; 1,68 (q, 2H) ; 3,92 (m, 1 H) ; 4,60 (q, 1 H) ; 5,07 (s, 2H) ; 5,49 (d, 1 H) ; 6,95-7 (massif, 3H) ; 7,26-7,62 (massif, 7H) ;7,87 (d, 1 H) ; 12,27 (s, 1 H).

$$[\alpha]_D^{20} = -72{,}6 \ (c = 1/CH_3OH).$$

**Exemple 6 (composé n˚40)**

**(2*S*)-2-[(2-hydroxy-3-phénylpropanoyl)amino]-*N*-{5-[2-(phénoxyméthyl)-phényl]-1,3-thiazol-2-yl}entanamide**

**6.1 acide 3-phényl-2-hydroxy-propionique**

A une suspension de 1,6g de phénylalanine dans 5,3 ml d'acide sulfurique (2,5N), on additionne goutte à goutte à 0˚C une solution de 0,829g de nitrite de sodium dans 4,2 ml d'eau. On agite le mélange réactionnel pendant

2 heures à 0°C puis 17 heures à température ambiante. On extrait le mélange réactionnel avec 2 fois 100 ml d'acétate d'éthyle. On lave les phases organiques rassemblées avec 100 ml de solution saturée de chlorure de sodium dans l'eau. On obtient après séchage 1,2 g de cristaux jaunes.
PF = 97°C

**6.2 2-[(2-hydroxy-3-phénylpropanoyl)amino]-*N*-{5-[2-(phénoxyméthyl)-phényl]-1,3-thiazol-2-yl}pentanamide**

On procède de la même manière que dans l'étape 1.8 de l'exemple 1 en remplaçant l'acide (2*S*)-2-hydroxy-3,3-diméthylbutanoïque par l'acide 3-phényl-2-hydroxy-propionique, obtenu à l'étape 6.1. On obtient 0,8 g de solide blanc.
PF = 86°C
RMN $^1$H $\delta$ en ppm (DMSO d 6) : 0,85 (t, 3H) ; 1,24 (m, 2H) ; 1,63 (m, 2H) ; 2,70 (m, 1H); 2,57 (m, 1H); 4,17 (m, 1H); 4,56 (q, 1 H) ; 5,08 (s, 2H) ; 6,94-7,63 (massif, 15H) ; 8,02 (m, 1 H) ; 12,25 (s, 1 H).

$[\alpha]_D^{20} = -28$ (c = 1/$CH_3OH$).

**Exemple 7 (composé n°70)**

**(2*S*)-2-{[(2*S*)-2-hydroxy-3,3-diméthylbutanoyl]amino}-*N*-(5-{2-[(4-pyridinyl-oxy)méthyl]phényl}-1,3-thiazol-2-yl)pentanamide**

**7.1 2-(2-amino-1,3-thiazol-5-yl)phénylméthanol**

A 29,86 g de 5-[2-(*tert*-butoxyméthyl)phényl]-*N*-diphénylméthylène)-1,3-thiazol-2-amine, préparée selon une méthode analogue à celle décrite aux étapes 1.1 à 1.4 de l'exemple 1, en solution dans 140 ml de méthanol, on additionne 70 ml d'une solution 3M d'acide chlorhydrique aqueux et on laisse 18 heures à température ambiante, puis on porte à reflux pendant 4 heures. On évapore le méthanol. On reprend le résidu pas une solution 6M d'acide chlorhydrique aqueux et on extrait à l'éther diéthylique. On amène la phase aqueuse à pH basique tout en la refroidissant et on l'extrait à l'acétate d'éthyle. On sèche la phase acétate d'éthyle sur sulfate de sodium anhydre et on concentre. On concrétise le résidu à l'éther diisopropylique pour obtenir 6 g d'un solide beige.
PF = 145°C

**7.2 *tert*-butyl-5-[2-(hydroxyméthyl)phényl]-1,3-thiazol-2-ylcarbamate**

A 6 g de 2-(2-amino-1,3-thiazol-5-yl)phénylméthanol, obtenu à l'étape 7.1, en solution dans 80 ml de 1,4-dioxane, on additionne successivement 1,17 g d'oxyde de magnésium, 29 ml d'une solution aqueuse 2M d'hydroxyde de sodium, puis à 0°C par portions 7,6 g de di-tert-butyldicarbonate ($BOC_2O$). On laisse 48 heures à température ambiante, puis on concentre le milieu, on le reprend par de l'eau et on l'extrait à l'acétate d'éthyle. On lave la phase organique par une solution à 5 % d'hydrogénosulfate de potassium, on la sèche sur sulfate de sodium anhydre et on la concentre. On chromatographie le résidu sur une colonne de gel de silice en éluant avec un mélange dichlorométhane/méthanol 99/1 (V/V) pour obtenir 2,3 g d'huile que l'on concrétise à l'éther diisopropylique.
PF = 180,7°C

**7.3 *tert*-butyl-5-{2-[(4-pyridinyloxy)méthyl]phényl}-1,3-thiazol-2-ylcarbamate**

A 5,24 g de triphénylphosphine en solution dans 60 ml de tétrahydrofurane, on additionne, par portions à 0°C, 4,15 g de diisopropylazodicarboxylate (DIAD). Après 30 minutes à environ 10°C on additionne par portions 1,96 g de 4-hydroxypyridine, on laisse 30 minutes à environ 10°C et on introduit 4,2 g de *tert*-butyl-5-[2-(hydroxyméthyl)phényl]-1,3-thiazol-2-yl carbamate, obtenu à l'étape 7.2. On laisse pendant 4 jours à température ambiante. On concentre le milieu, on le reprend par une solution saturée de carbonate de sodium et on extrait au dichlorométhane. On sèche la phase organique sur sulfate de sodium anhydre et on la concentre. On chromatographie le résidu sur une colonne de gel de silice en éluant avec un mélange de polarité croissante dichlorométhane/méthanol 99/1 (VN) jusqu'à dichlorométhane/méthanol, 90/10 (VN) pour obtenir 1 g d'huile.
RMN $^1$H $\delta$ en ppm (DMSO d 6) : 1,52 (s, 9H) ; 5,27 (s, 2H) ; 6,10 (q, 2H) ; 7,05 (m, 1 H) ; 7,41-7,54 (massif, 6H) ; 11,60 (s, 1 H).

**7.4 5-{2-[(4-pyridinyloxy)méthyl]phényl}-1,3-thiazolamine**

A 1 g de *tert*-butyl-5-{2-[(4-pyridinyloxy)méthyl]phényl}-1,3-thiazol-2-yl carbamate, obtenu à l'étape 7.3, dans 20 ml de dichlorométhane, on additionne 20 ml d'une solution de 4M d'acide chlorhydrique gaz dans l'acétate d'éthyle. On laisse 4 heures à température ambiante. On concentre le milieu réactionnel et on l'amène à pH basique par une solution à 5 % d'hydrogénosulfate de sodium. On filtre le précipité formé et on chromatographie sur une colonne de gel de silice en éluant avec un mélange dichlorométhane/méthanol 95/5 (VN) pour obtenir 0,58 g d'une mousse beige.
RMN $^1H$ $\delta$ en ppm (DMSO d 6) : 5,25 (s, 2H) ; 6,10 (d, 2H) ; 6,98-7,55 (massif, 9 H).

**7.5 (2*S*)-2-{[(2*S*)-2-hydroxy-3,3-diméthylbutanoyl]amino}-*N*-(5-{2-[(4-pyridinyloxy)méthyl]phényl}-1,3-thiazol-2-yl)pentanamide**

On procède de la même manière que dans les étapes 1.6 à 1.8 de l'exemple 1 en remplaçant la 5-{2-[(phénoxy) méthyl]phényl}-1,3-thiazol-2-amine par la 5-{2-[(4-pyridinyloxy)méthyl]phényl}-1,3-thiazolamine, obtenu à l'étape 7.4. On obtient 0,4 g sous forme de cristaux.
PF = 112,7°C
RMN $^1H$ $\delta$ en ppm (DMSO d6) : 0,89 (t, 3H) ; 0,92 (s, 9H) ; 1,34 (m, 2H) ; 1,68 (q, 2H) ; 3,58 (d, 1 H) ; 4,61 (q, 1 H) ; 5,24 (s, 2H) ; 5,61 (d, 1 H) ; 6,09 (d, 2H) ; 7,04 (m, 1 H) ; 7,41-7,53 (massif, 6H) ; 7,80 (d, 1 H) ; 12,36 (s, 1 H).

Les composés n°61 ((2*S*)-*N*-(5-{2-[(3-cyanophénoxy)méthyl]phényl}-1,3-thiazol-2-yl)-2-{[(2*S*)-2-hydroxy-3,3-diméthylbutanoyl]amino}pentanamide) et n°69 ((2*S*)-*N*-(5-{2-[(2-cyanophénoxy)méthyl]phényl}-1,3-thiazol-2-yl)-2-{[(2*S*)-2-hydroxy-3,3-diméthylbutanoyl]amino}pentanamide) peuvent être préparés selon la méthode décrite à l'exemple 7 en remplaçant la 4-hydroxypyridine par respectivement le 3-cyanophénol ou le 2-cyanophénol.

Le tableau suivant illustre les structures chimiques et les propriétés physiques de quelques composés de l'invention.
Dans ce tableau :

- PF(°C) représente le point de fusion du composé en degré Celsius ;
- [$\alpha_D$] (c=1, $CH_3OH$) représente le pouvoir rotatoire du composé à la concentration de 1 g/l dans le méthanol ;
- (S) ou (R) dans les colonnes « $R_3$ » et « $R_5$, $R_{5'}$ » indiquent la stéréochimie des carbones asymétriques, portant respectivement $R_3$ ou $R_5$, dans la formule (I). Pour le carbone portant $R_5$, l'indication (S) ou (R) ne concerne pas le cas où $R_5$ et $R_{5'}$ forment ensemble un groupe oxo ou oxime.

Les composés décrits dans ce tableau ont été préparés selon les méthodes décrites précédemment.

**Tableau**

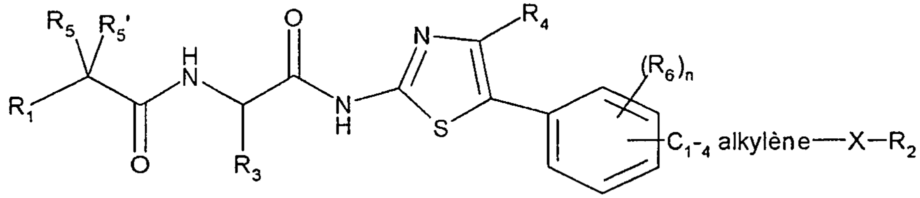

(I)

| N° | $R_1$ | $R_3$ | $R_4$ | $R_5$, $R_{5'}$ | 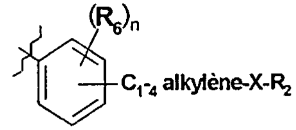 | PF (°C) | $[\alpha_D]$ (c=1, $CH_3OH$) |
|---|---|---|---|---|---|---|---|
| 1. | 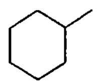 | $CH_3(CH_2)_2$-(S) | H | OH, H (R) | 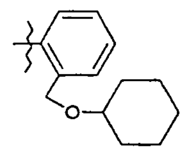 | 120,5 | -51 |
| 2. | 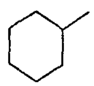 | $CH_3(CH_2)_2$-(S) | H | OH, H (S) | 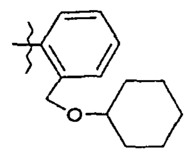 | 106 | -81,8 |
| 3. | 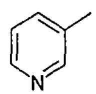 | $CH_3(CH_2)_2$-(S) | H | H, H | 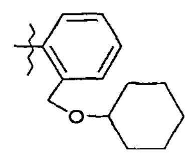 | 110 | -109,5 |

(suite)

| N° | $R_1$ | $R_3$ | $R_4$ | $R_5$, $R_{5'}$ | 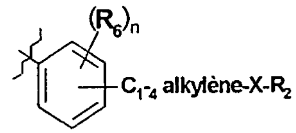 | PF (°C) | $[\alpha_D]$ (c=1, $CH_3OH$) |
|---|---|---|---|---|---|---|---|
| 4. | $(CH_3)_2CHCH_2$- | $CH_3(CH_2)_2$-(S) | H | OH, H | 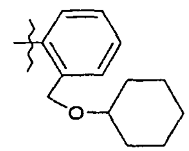 | 85 | -62,9 |
| 5. | 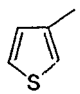 | $CH_3(CH_2)_2$-(S) | $CH_3$ | H, H | 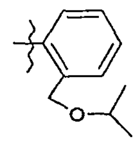 | 72 | -65 |
| 6. | 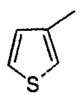 | $CH_3(CH_2)_2$-(S) | H | H, H | 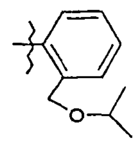 | 51 | -89 |
| 7. | 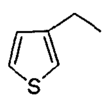 | $CH_3(CH_2)_2$-(S) | H | H, H | 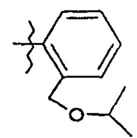 | 101 | -71 |
| 8. | 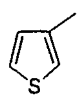 | $CH_3(CH_2)_2$-(S) | H | H, H | 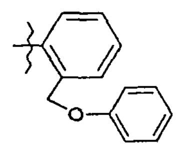 | 74 | -78,9 |

(suite)

| N° | $R_1$ | $R_3$ | $R_4$ | $R_5$, $R_{5'}$ | 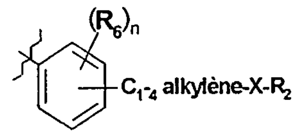 | PF (°C) | [$\alpha_D$] (c=1, $CH_3OH$) |
|---|---|---|---|---|---|---|---|
| 9. | $(CH_3)_3C$- | $CH_3(CH_2)_2$-(S) | H | OH, H (S) | 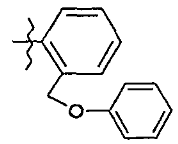 | 84 | -81,8 |
| 10. | 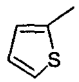 | $CH_3CH_2$- (S) | H | H, H | 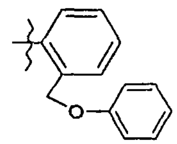 | 76,5 | -100,2 |
| 11. | $(CH_3)_3C$- | $CH_3(CH_2)_2$-(S) | H | OH, H (S) | 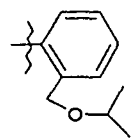 | 63 | -87 |
| 12. | 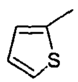 | $CH_3(CH_2)_2$-(S) | H | H, H | 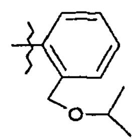 | 62 | -88 |
| 13. | $(CH_3)_3C$- | $CH_3(CH_2)_2$-(S) | H | H, H | 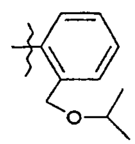 | 77 | -81 |

(suite)

| N° | $R_1$ | $R_3$ | $R_4$ | $R_5$, $R_{5'}$ | 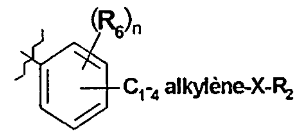 | PF (°C) | $[\alpha_D]$ (c=1, $CH_3OH$) |
|---|---|---|---|---|---|---|---|
| 14. | $(CH_3CH_2)(CH_3)$ CH- | $CH_3(CH_2)_2$-(S) | H | =O | 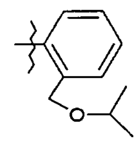 | 112 | -66 |
| 15. | $(CH_3)_3C$- | $CH_3(CH_2)_2$-(S) | H | OH, H (S) | 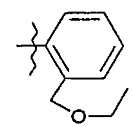 | 148 | -87 |
| 16. | 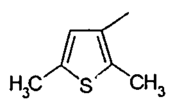 | $CH_3(CH_2)_2$-(S) | H | H, H | 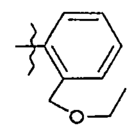 | 84 | -103 |
| 17. | $(CH_3)_3C$- | $CH_3(CH_2)_2$-(S) | H | OH, H (S) | 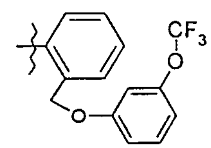 | 114 | -75 |
| 18. | $(CH_3)_3C$- | $CH_3(CH_2)_2$-(S) | H | OH, H (S) | 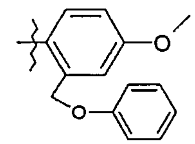 | 117 | -78,3 |

(suite)

| N° | $R_1$ | $R_3$ | $R_4$ | $R_5$, $R_{5'}$ | 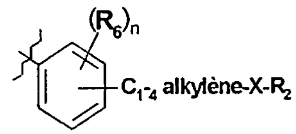 | PF (°C) | $[\alpha_D]$ (c=1, $CH_3OH$) |
|---|---|---|---|---|---|---|---|
| 19. | $(CH_3)_3C$- | $CH_3(CH_2)_2$-(S) | H | OH, H (S) | 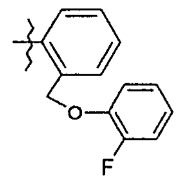 | 92 | -77,7 |
| 20. | $(CH_3)_3C$- | $CH_3(CH_2)_2$-(S) | H | OH, H (S) | 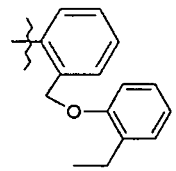 | 107 | -88,7 |
| 21. | 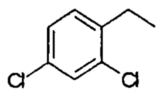 | $CH_3(CH_2)_2$-(S) | H | H, H | 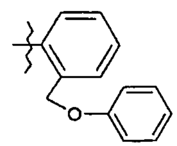 | 82 | -31,3 |
| 22. | 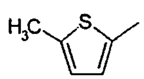 | $CH_3(CH_2)_2$-(S) | H | H, H | 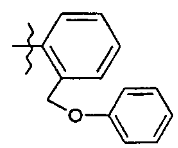 | 81 | -91,7 |
| 23. | $(CH_3)_3C$- | $CH_3(CH_2)_2$-(S) | H | OH, H (S) | 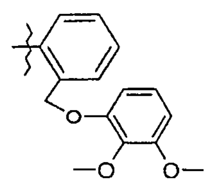 | 99 | -71 |

(suite)

| N° | $R_1$ | $R_3$ | $R_4$ | $R_5$, $R_{5'}$ | 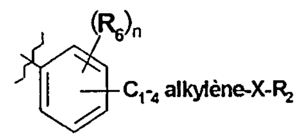 | PF (°C) | $[\alpha_D]$ (c=1, $CH_3OH$) |
|---|---|---|---|---|---|---|---|
| 24. | $(CH_3)_3C$- | $CH_3(CH_2)_2$-(S) | H | OH, H (S) | 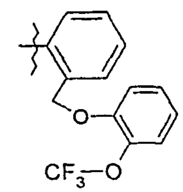 | 66 | -64,4 |
| 25. | $(CH_3)_3C$- | $CH_3(CH_2)_2$-(S) | H | OH, H (S) | 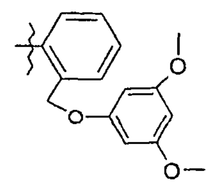 | 66,5 | -65,4 |
| 26. | $(CH_3)_3C$- | $CH_3(CH_2)_2$-(S) | H | OH, H (S) | 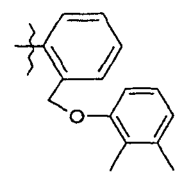 | 88 | -80,6 |
| 27. | $(CH_3)_3C$- | $CH_3(CH_2)_2$-(S) | H | OH, H (S) | 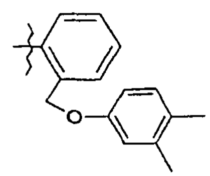 | 89 | -74,6 |

(suite)

| N° | $R_1$ | $R_3$ | $R_4$ | $R_5$, $R_{5'}$ | 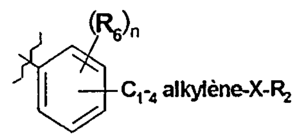 | PF (°C) | $[\alpha_D]$ (c=1, $CH_3OH$) |
|---|---|---|---|---|---|---|---|
| 28. | $(CH_3)_3C$- | $CH_3(CH_2)_2$-(S) | H | OH, H (S) | 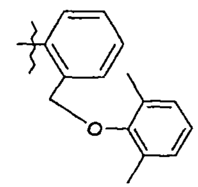 | 87 | -81,7 |
| 29. | $(CH_3)_3C$- | $CH_3(CH_2)_2$-(S) | H | OH, H (S) | 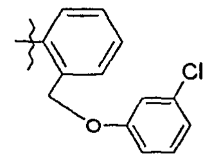 | 169 | -83,5 |
| 30. | $(CH_3)_3C$- | $CH_3(CH_2)_2$-(S) | H | OH, H (S) | 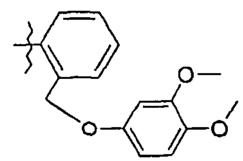 | 81,5 | -63 |
| 31. | $(CH_3)_3C$- | $CH_3(CH_2)_2$-(S) | H | OH, H (S) | 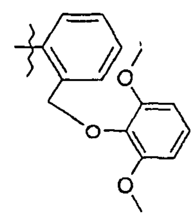 | 69,5 | -63,2 |

(suite)

| N° | $R_1$ | $R_3$ | $R_4$ | $R_5$, $R_{5'}$ | 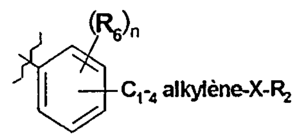 | PF (°C) | [$\alpha_D$] (c=1, $CH_3OH$) |
|---|---|---|---|---|---|---|---|
| 32. | $(CH_3)_3C$- | $CH_3(CH_2)_2$-(S) | H | OH, H (S) | 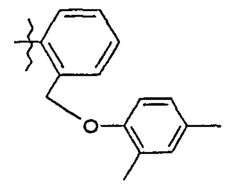 | 86 | -70,5 |
| 33. | $(CH_3)_3C$- | $CH_3(CH_2)_2$-(S) | H | OH, H (S) | 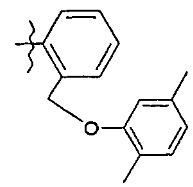 | 87 | -75,4 |
| 34. | $(CH_3)_3C$- | $CH_3(CH_2)_2$-(S) | $CH_3$- | OH, H (S) | 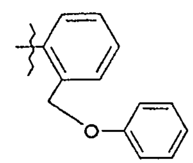 | 99 | -74,4 |
| 35. | $(CH_3)_3C$- | $CH_3(CH_2)_2$-(R) | H | OH, H (S) | 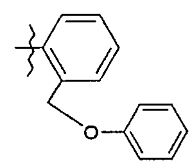 | 78,5 | +46,7 |

(suite)

| N° | $R_1$ | $R_3$ | $R_4$ | $R_5$, $R_{5'}$ | 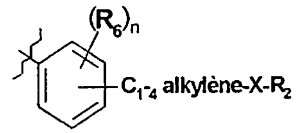 | PF (°C) | [$\alpha_D$] (c=1, $CH_3OH$) |
|---|---|---|---|---|---|---|---|
| 36. | $(CH_3)_3C$- | $CH_3(CH_2)_2$-(S) | H | OH, H (S) | 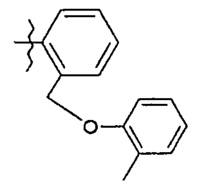 | 96,5 | -66,7 |
| 37. | $(CH_3)_2CH$- | $CH_3(CH_2)_2$-(S) | H | OH, H (R) | 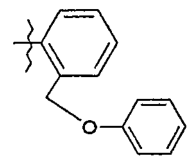 | 141 | -59,6 |
| 38. | $(CH_3)_3C$- | $CH_3(CH_2)_2$-(S) | H | OH, H (S) | 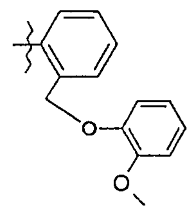 | 85 | -78 |
| 39. | $(CH_3)_3C$- | $CH_3(CH_2)_2$-(S) | H | OH, H (R) | 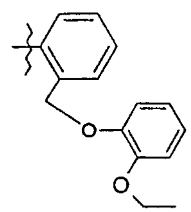 | 88 | -74,4 |

(suite)

| N° | $R_1$ | $R_3$ | $R_4$ | $R_5$, $R_{5'}$ | 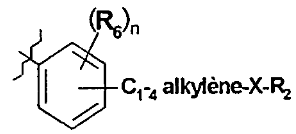 | PF (°C) | $[\alpha_D]$ (c=1, $CH_3OH$) |
|---|---|---|---|---|---|---|---|
| 40. | 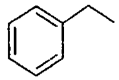 | $CH_3(CH_2)_2$-(S) | H | OH, H | 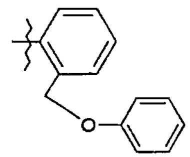 | 86 | -28 |
| 41. | $(CH_3)_3C$- | $CH_3(CH_2)_2$-(S) | H | OH, H | 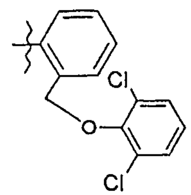 | 126 | -62,5 |
| 42. | $(CH_3CH_2)_2CH$- | $CH_3(CH_2)_2$-(S) | H | OH, H (R) | 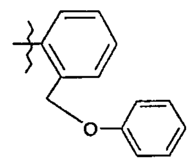 | 67,5 | -41,5 |
| 43. | $(CH_3CH_2)_2CH$- | $CH_3(CH_2)_2$-(S) | H | OH, H (S) | 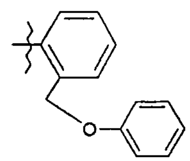 | 122,5 | -72,6 |
| 44. | $(CH_3)_3C$- | $CH_3(CH_2)_2$-(S) | H | OH, H (S) | 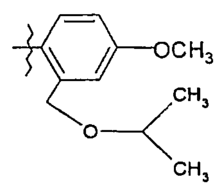 | 79 | -77,9 |

(suite)

| N° | $R_1$ | $R_3$ | $R_4$ | $R_5$, $R_{5'}$ | 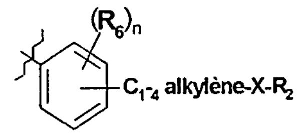 | PF (°C) | $[\alpha_D]$ (c=1, $CH_3OH$) |
|---|---|---|---|---|---|---|---|
| 45. | $(CH_3)_3C$- | $CH_3(CH_2)_2$-(S) | H | OH, H (S) | 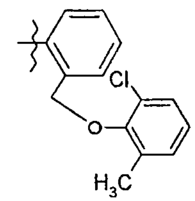 | 96 | -67,3 |
| 46. | $(CH_3)_3C$- | $CH_3(CH_2)_2$-(S) | H | OH, H (S) | 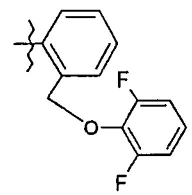 | 63,5 | -75 |
| 47. | $(CH_3)_3C$- | $CH_3(CH_2)_2$-(S) | H | OH, H (S) | 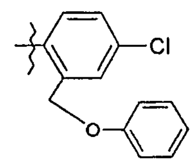 | 93,5 | -80,5 |
| 48. | $(CH_3)_3C$- | $CH_3(CH_2)_2$-(S) | H | OH, H (S) | 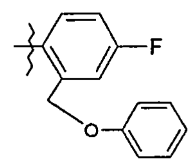 | 75,5 | -75,3 |
| 49. | $(CH_3)_2CH$- | $CH_3(CH_2)_2$-(S) | H | OH, H (S) | 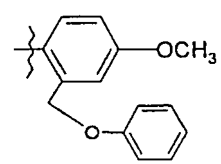 | 159,5 | -67,8 |

(suite)

| N° | $R_1$ | $R_3$ | $R_4$ | $R_5$, $R_{5'}$ | 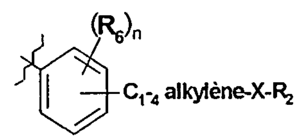 | PF (°C) | [$\alpha_D$] (c=1, $CH_3OH$) |
|---|---|---|---|---|---|---|---|
| 50. | $(CH_3)_3C$- | $CH_3(CH_2)_2$-(S) | H | OH, H (S) | 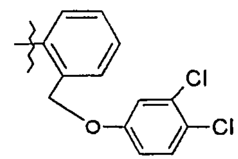 | | -72,1 |
| 51 | $(CH_3)_2CHCH_2$- | $CH_3(CH_2)_2$-(S) | H | OH, H (S) | 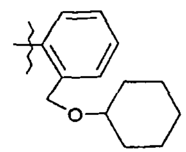 | 158 | -70,2 |
| 52. | $(CH_3)_3C$- | $CH_3(CH_2)_2$-(S) | H | OH, H (S) | 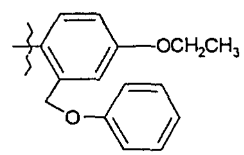 | 81 | -75,8 |
| 53. | $(CH_3)_2CH$- | $CH_3(CH_2)_2$-(S) | H | OH, H (S) | 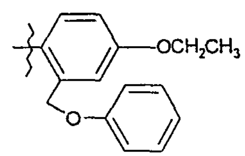 | 107,8 | -66,7 |
| 54. | $(CH_3)_3C$- | $CH_3(CH_2)_2$-(S) | H | OH, H (S) | 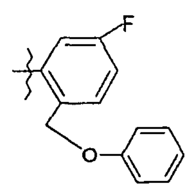 | 105,4 | -78,4 |

(suite)

| N° | $R_1$ | $R_3$ | $R_4$ | $R_5$, $R_5$' | 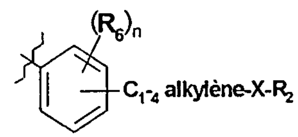 | PF (°C) | $[\alpha_D]$ (c=1, $CH_3OH$) |
|---|---|---|---|---|---|---|---|
| 55. | $(CH_3)_3C$- | $CH_3(CH_2)_2$-(S) | H | OH, H (S) | 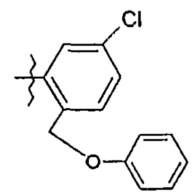 | 120,7 | -77,2 |
| 56. | $(CH_3)_2CH$- | $CH_3(CH_2)_2$-(S) | H | OH, H (S) | 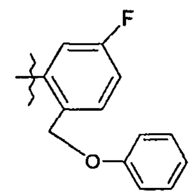 | 61,4 | -48,6 |
| 57. | $(CH_3)_3C$- | $CH_3(CH_2)_2$-(S) | H | OH, H (S) | 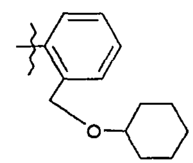 | 88 | -84,9 |
| 58. | $(CH_3)_3C$- | $CH_3$-(S) | H | OH, H (S) | 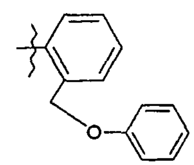 | 118,6 | -106,9 |
| 59. | $(CH_3)_3C$- | $CH_3(CH_2)_2$-(S) | H | OH, H (S) | 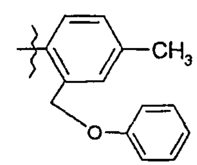 | 92,6 | -88,5 |

(suite)

| N° | $R_1$ | $R_3$ | $R_4$ | $R_5$, $R_{5'}$ | 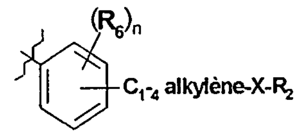 | PF (°C) | $[\alpha_D]$ (c=1, $CH_3OH$) |
|---|---|---|---|---|---|---|---|
| 60. | $(CH_3)_2CH$- | $CH_3(CH_2)_2$-(S) | H | OH, H (S) | 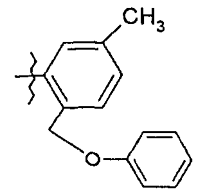 | 125,5 | -85 |
| 61. | $(CH_3)_3C$- | $CH_3(CH_2)_2$-(S) | H | OH, H (S) | 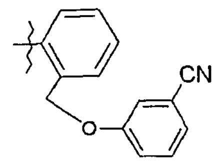 | 162,6 | -104,5 |
| 62. | $(CH_3)_3C$- | $CH_3(CH_2)_2$-(S) | H | OH, H (S) | 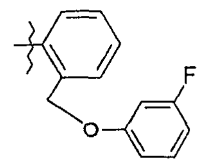 | 160,2 | -71,5 |
| 63. | $(CH_3)_2CH$- | $CH_3(CH_2)_2$-(S) | H | OH, H (S) | 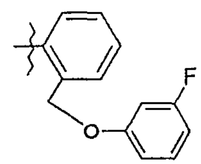 | 130,7 | -71,6 |

(suite)

| N° | $R_1$ | $R_3$ | $R_4$ | $R_5$, $R_{5'}$ | 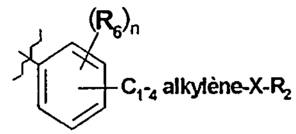 | PF (°C) | [$\alpha_D$] (c=1, $CH_3OH$) |
|---|---|---|---|---|---|---|---|
| 64. | $(CH_3)_3C$- | $CH_3(CH_2)_2$-(S) | H | OH, H (S) | 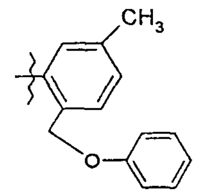 | 170 | -78,4 |
| 65. | $(CH_3)_2CH$- | $CH_3(CH_2)_2$-(S) | H | OH, H (S) | 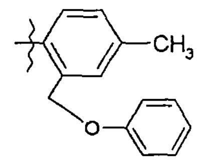 | 135,5 | -72,2 |
| 66. | $(CH_3)_3C$- | $CH_3$-(S) | H | OH, H (S) | 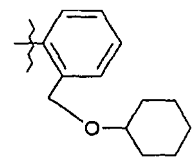 | 104,8 | -108,6 |
| 67. | $(CH_3)_3C$- | $CH_3(CH_2)_2$-(S) | H | OH, H (S) | 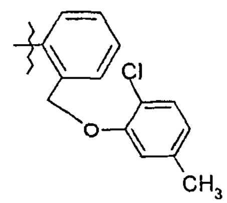 | 82 | -72 |

(suite)

| N° | $R_1$ | $R_3$ | $R_4$ | $R_5$, $R_{5'}$ | 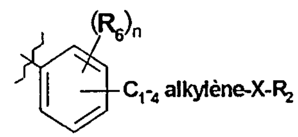 | PF (°C) | $[\alpha_D]$ (c=1, $CH_3OH$) |
|---|---|---|---|---|---|---|---|
| 68. | $(CH_3)_3C$- | $CH_3(CH_2)_2$-(S) | H | OH, H (S) | 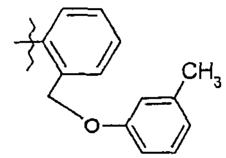 | 80,6 | -64,7 |
| 69 | $(CH_3)_3C$- | $CH_3(CH_2)_2$-(S) | H | OH, H (S) | 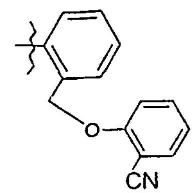 | 99 | -84,6 |
| 70 | $(CH_3)_3C$- | $CH_3(CH_2)_2$-(S) | H | OH, H (S) | 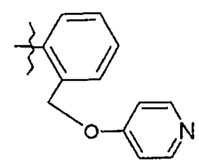 | 112,7 | -63,3 |
| 71 | $(CH_3)_3C$- | $CH_3(CH_2)_2$-(S) | H | OH, H (S) | 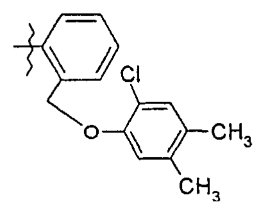 | 85 | -68 |

(suite)

| N° | $R_1$ | $R_3$ | $R_4$ | $R_5$, $R_{5'}$ | 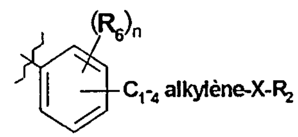 | PF (°C) | [$\alpha_D$] (c=1, $CH_3OH$) |
|---|---|---|---|---|---|---|---|
| 72. | $(CH_3)_3C$- | $CH_3(CH_2)_2$-(S) | H | OH, H (S) | 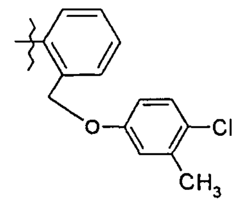 | 83,4 | -56,2 |
| 73. | $(CH_3)_3C$- | $CH_3(CH_2)_2$-(S) | H | OH, H (S) | 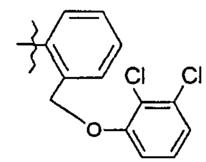 | 71,1 | -69,81 |
| 74. | $(CH_3)_3C$- | $CH_3(CH_2)_2$-(S) | H | OH, H (S) | 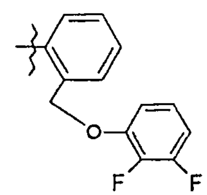 | 82 | -76,1 |
| 75. | $(CH_3)_3C$- | $CH_3(CH_2)_2$- (S) | 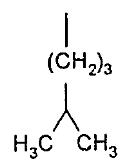 | OH, H (S) | 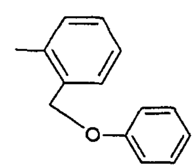 | 96,2 | -56,3 |

Les composés de l'invention ont fait l'objet d'essais pharmacologiques qui ont montré leur intérêt comme substances actives en thérapeutique.

Ils ont en particulier été testés quant à leurs effets d'inhibition de la production du peptide β-amyloïde (β-A4).

Le peptide β-amyloïde (β-A4) est un fragment d'une protéine précurseur plus importante appelée APP (Amyloid Precursor Protein). Cette dernière est produite et présente dans différentes cellules de tissu animal ou humain. Au niveau cérébral, son clivage par des enzymes de type protéase conduit à la formation du peptide β-A4 qui s'accumule sous forme de plaque amyloïde. Les deux protéases responsables de la production du peptide amyloide sont connues sous le nom de beta et gamma-secrétases (Wolfe MS, Secretase targets for Alzheimer's disease: identification and therapeutic potential, J. Med. Chem., 2001 Jun 21;44(13), 2039-60).

Or il a été démontré que ce dépôt progressif du peptide β-A4 est neurotoxique et pourrait jouer un rôle important dans la maladie d'Alzheimer.

Ainsi, les composés de la présente invention, en tant qu'inhibiteur de la production du peptide β-amyloïde (β-A4) par inhibition de la gamma protéase, peuvent être utilisés dans le traitement de pathologies comme la démence sénile, la maladie d'Alzheimer, le syndrome de Down, la maladie de Parkinson, l'angiopathie amyloïde, les désordres cérébro-vasculaires, les démences fronto-temporales et la maladie de Pick, les démences post-traumatiques, les pathologies liées à des processus de neuro-inflamation, la maladie de Huntington et le syndrome de Korsakov.

Les tests ont été réalisés selon le protocole décrit ci-après.

Pour l'essai cellulaire β amyloïde, la lignée CHO-K1 coexprimant le CT100 de APP et PS1 M146L clone 30-12 est utilisée. La lignée cible l'inhibition de gamma secrétase. La préséniline est liée à l'activité gamma-secrétase (Wolfe MS, Haass C., The Role of presenilins in gamma-secretase activity, J. Biol. Chem., 2001 Feb 23, 276(8), 5413-6) et sa coexpression avec la protéine amyloïde ou son fragment N-terminal entraîne une augmentation de secrétion du peptide A1-42 (β-A4) générant ainsi un outil pharmacologique permettant d'évaluer l'inhibition par les composés de formule (I) de la production du peptide β-A4. L'ensemencement des plaques de culture de 96 puits est réalisé à raison de $1x10^5$ cellules par puits dans 150μl de milieu d'incubation. La présence d'un pourcentage minimal (1,3% final) de sérum permet l'adhésion cellulaire au plastique après 2-3 heures d'incubation à 37˚C, en présence de 5% $CO_2$. Les produits (15μl) sont testés à 10μM DMSO 1 % final et sont incubés durant 24-25h à 37˚C en présence de 5% $CO_2$ et de 100% d'humidité. Après cette incubation de 24-25h, les surnageants cellulaires (100μl) sont transférés dans les plaques ELISA, traitées avec l'anticorps de capture 6E10 (6E10, épitope : aa1-17, INTERCHIM/SENETEK 320-10), pour déterminer le taux de peptides amyloïdes sécrété par les cellules en présence de composés selon l'invention. Une gamme de peptide contrôle, « peptide 1-40 », synthétique à 5 et 10 ng/ml est traitée parallèlement. Les plaques ELISA sont incubées pendant une nuit à 4˚C.

La quantité de peptide fixé est détectée de façon indirecte en présence d'un compétiteur correspondant au peptide tronqué, le peptide 1-28 couplé à la biotine qui est ensuite détectée par la streptavidine couplée à la phosphatase alcaline. Le substrat, le p-Nitrophényl Phosphate (pNPP *FAST* p-Nitrophényl Phosphate, Sigma N2770), donne un produit de réaction soluble jaune lisible à 405nm. La réaction est stoppée avec une solution d'EDTA 0,1M. Pour cela, après fixation du peptide amyloïde dans la plaque ELISA, 50μl de peptide 1-28 biotinylé sont ajoutés aux 100 μl de surnageant cellulaire et incubés 30 minutes à température ambiante. Les plaques ELISA sont ensuite lavées 3 fois. Après séchage par inversion sur papier absorbant, 100μl de streptavidine-Alcaline Phosphatase (Interchim/Jackson ImmunoResearch Laboratories 016-050-084), sont ajoutés par puits et incubés 1 heure à température ambiante. Les plaques sont à nouveau lavées puis le substrat de la phosphatase alcaline (pNPP 1mg/ml) est ajouté à raison de 100μl par puits. Après une incubation de 30 minutes à température ambiante, la réaction est stoppée par ajout de 100μl par puits d'EDTA 0,1M et la lecture est effectuée à 405nm.

Les composés de formule (I) selon l'invention ont montré une CE50 (concentration effectrice à 50%) inférieure à 500 nM. Notamment, le composé n˚ 70 du tableau a montré une CE5O égale à 295 nM. Les composés de formule (I) selon l'invention ont montré plus particulièrement une CE5O inférieure à 100 nM.

Les résultats des tests biologiques montrent que les composés sont des inhibiteurs de la formation du peptide β-amyloïde (β-A4).

Ainsi, ces composés peuvent être employés dans le traitement des pathologies dans lesquelles un inhibiteur de la formation du peptide β-amyloïde (β-A4) apporte un bénéfice thérapeutique. Notamment de telles pathologies sont la démence sénile, la maladie d'Alzheimer, le syndrome de Down, la maladie de Parkinson, l'angiopathie amyloïde, les désordres cérébro-vasculaires, les démences fronto-temporales et la maladie de Pick, les démences post-traumatiques, les pathologies liées à des processus de neuro-inflamation, la maladie de Huntington et le syndrome de Korsakov.

L'utilisation des composés selon l'invention, à l'état de base, de sel, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition de ce dernier à un acide pharmaceutiquement acceptable ou encore un hydrate ou un solvat du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel pharmaceutiquement acceptable, un hydrate ou un solvat dudit composé, et éventuellement un ou plusieurs excipients pharmaceutiquement acceptables. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

La présente invention selon un autre de ses aspects, concerne également une méthode de traitement des pathologies ci-dessus indiquées qui comprend l'administration d'un composé selon l'invention, d'un sel pharmaceutiquement acceptable, d'un solvat ou d'un hydrate dudit composé.

## Revendications

**1.** Composé répondant à la formule générale (I) :

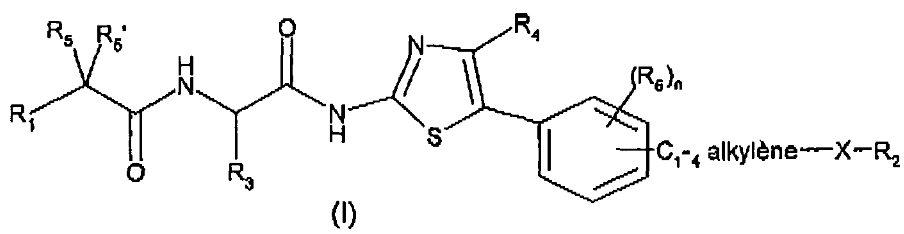

(I)

dans laquelle,

X représente un atome d'oxygène ou de soufre ;

$R_1$ représente un groupe $C_{1-10}$ alkyle éventuellement substitué par un $C_{3-7}$ cycloalkyle, un phényle, un thiényle ;

ou $R_1$ représente un groupe $C_{3-7}$ cycloalkyle, thiényle, pyridinyle, pyrimidinyle ;
les groupes thiényle étant éventuellement substitués par un à 3 groupes $C_{1-3}$ alkyle ; le groupe phényle étant éventuellement substitué par un à 5 atomes d'halogène ou groupes $C_{1-3}$ alkyle, $C_{1-3}$ alcoxy, $C_{1-3}$ fluoroalkyle, $C_{1-3}$ fluoroalcoxy ;
$R_2$ représente un groupe $C_{1-6}$ alkyle éventuellement substitué par un groupe $C_{3-7}$ cycloalkyle, phényle, $C_{1-3}$ alcoxy, hydroxy ; ou $R_2$ représente un groupe $C_{3-7}$ cycloalkyle, pipéridinyle, phényle ou pyridinyle ;
les groupes $C_{3-7}$ cycloalkyle et pipéridinyle étant éventuellement substitués par un ou plusieurs groupes $C_{1-3}$ alkyle, $C_{1-3}$ alcoxy, hydroxy, $C_{1-3}$ fluoroalkyle, $C_{1-3}$ fluoroalcoxy ;
les groupes phényle et pyridinyle étant éventuellement substitués par un ou plusieurs atomes d'halogène ou groupes $C_{1-3}$ alkyle, $C_{1-3}$ alcoxy, hydroxy, $C_{1-3}$ fluoroalkyle, $C_{1-3}$ fluoroalcoxy ;
$R_3$ représente un atome d'hydrogène ou un groupe $C_{1-6}$ alkyle éventuellement substitué par un groupe $C_{3-7}$ cycloalkyle ;
$R_4$ représente un atome d'hydrogène ou un groupe $C_{1-6}$ alkyle ;
$R_5$ et $R_{5'}$ représentent, indépendamment l'un de l'autre, un atome d'hydrogène, d'halogène, un groupe hydroxy, $C_{1-3}$ alkyle; ou $R_5$ et $R_{5'}$ forment ensemble un groupe oxo ou oxime tel que :

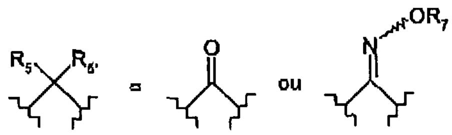

où $R_7$ représente un atome d'hydrogène ou un $C_{1-3}$ alkyle ;
n représente un nombre entier allant de 0 à 3 ; et
$R_6$ représente, indépendamment l'un de l'autre lorsque n = 2 ou 3, un atome d'hydrogène, d'halogène, un groupe hydroxy, $C_{1-3}$ alkyle, $C_{1-3}$ alcoxy, $C_{1-3}$ fluoroalkyle ou $C_{1-3}$ fluoroalcoxy ;
à l'état de base, de sel d'addition à un acide, d'hydrate ou de solvat.

**2.** Composé répondant à la formule générale (I) :

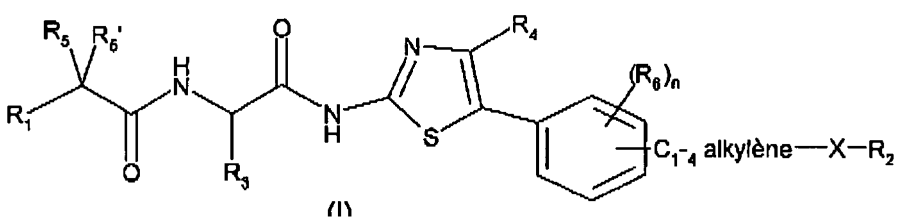

(I)

dans laquelle X, $R_1$, $R_3$, $R_4$, $R_5$, $R'_5$ et $R_6$ sont tels que définis dans la revendication 1 et $R_2$ représente un groupe $C_{1-6}$ alkyle substitué par un groupe phényle ou $R_2$ représente un groupe phényle ou pyridinyle, lesdits groupes phényle et pyridinyle étant substitués par un ou plusieurs groupes CN.

**3.** Composé de formule (I) selon la revendication 1 **caractérisé en ce que**
X représente un atome d'oxygène ou de soufre ;
$R_1$ représente un groupe $C_{1-5}$ alkyle, éventuellement substitué par un phényle, un thiényle ; ou $R_1$ représente un groupe $C_{3-7}$ cycloalkyle, un groupe thiényle, pyridinyle ; les groupes thiényle étant éventuellement substitués par un ou deux groupes $C_{1-3}$ alkyle ; le groupe phényle étant éventuellement substitué par un ou deux atomes d'halogène;
$R_2$ représente un groupe $C_{1-6}$ alkyle, $C_{3-7}$ cycloalkyle, phényle ou pyridinyle ;
le groupe phényle étant éventuellement substitué par un à trois groupes $C_{1-3}$ alkyle, $C_{1-3}$ alcoxy, hydroxy, fluoroalcoxy, ou atomes d'halogène;
$R_3$ représente un groupe $C_{1-6}$ alkyle ;
$R_4$ représente un atome d'hydrogène ou un groupe $C_{1-6}$ alkyle;
$R_5$ et $R_{5'}$ représentent, indépendamment l'un de l'autre, un atome d'hydrogène, un hydroxy; ou $R_5$ et $R_{5'}$ forment ensemble un groupe oxo ;
$R_6$ représente un atome d'hydrogène, d'halogène, un $C_{1-3}$ alkyle, un $C_{1-3}$ alcoxy ; et
n est égal à 0 ou 1;

à l'état de base, de sel d'addition à un acide, d'hydrate ou de solvat.

**4.** Composé selon la revendication 2, **caractérisé en ce que** $R_2$ représente un groupe phényle, ledit groupe phényle étant substitué par un à trois groupes CN, et X, $R_1$, $R_3$, $R_4$, $R_5$, $R'_5$ et $R_6$ sont tels que définis dans la revendication 3.

**5.** Composé de formule (I) selon la revendication 1 ou 3, **caractérisé en ce que :**

X représente un atome d'oxygène;
$R_1$ représente un méthyle, éthyle, 1-méthyléthyle, 1,1-diméthyléthyle, propyle, 1-méthylpropyle, 2-méthylpropyle ou 1-éthylpropyle, éventuellement substitué par un phényle ou par un thiényle ; ou $R_1$ représente un cyclohexyle, thiényle ou pyridinyle ; les groupes thiényle étant éventuellement substitués par un ou deux groupes méthyle ; le groupe phényle étant éventuellement substitué par un ou deux atomes de chlore ou de fluor ;
$R_2$ représente un groupe éthyle, 1-méthyléthyle, cyclohexyle, phényle ou pyridinyle ;
le groupe phényle étant éventuellement substitué par un à trois groupes méthyle, éthyle, méthoxy, éthoxy, hydroxy, trifluorométhoxy, ou atomes de chlore ou de fluor ;
$R_3$ représente un groupe méthyle, éthyle ou propyle;
$R_4$ représente un atome d'hydrogène ou un groupe méthyle ou 4-méthylpentyl ;
$R_5$ et $R_{5'}$ représentent, indépendamment l'un de l'autre, un atome d'hydrogène, un hydroxy; ou $R_5$ et $R_{5'}$ forment ensemble un groupe oxo ;
$R_6$ représente un atome d'hydrogène, de chlore ou de fluor, un méthyle, un méthoxy ou un éthoxy ;
n est égal à 0 ou 1 ; et
le groupe $C_{1-4}$ alkylène est un méthylène;
à l'état de base, de sel d'addition à un acide, d'hydrate ou de solvat.

**6.** Composé selon la revendication 2 ou 4, **caractérisé en ce que** $R_2$ représente un groupe phényle, ledit groupe phényle étant substitué par un à trois groupes CN et X, $R_1$, $R_3$, $R_4$, $R_5$, $R'_5$ et $R_6$ sont tels que définis dans la revendication 5.

**7.** Procédé de préparation d'un composé de formule (I) selon l'une quelconque des revendications 1 à 6, par couplage peptidique d'une amine de formule (XI)

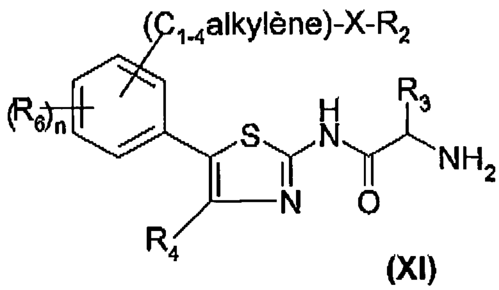

(XI)

avec un acide de formule (XII)

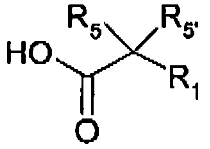

(XII)

dans lesquelles X, $R_1$, $R_2$, $R_3$, $R_4$, $R_5$, $R_{5'}$, $R_6$ et n sont tels que défini dans la formule (I) selon la revendication 1.

**8.** Composition pharmaceutique contenant au moins un composé de formule (I) selon l'une quelconque des revendications 1 à 6, à l'état de base, de sel, d'hydrate ou de solvat pharmaceutiquement acceptable et éventuellement un ou plusieurs excipients pharmaceutiquement acceptables.

**9.** Composé de formule (I) selon l'une quelconque des revendications 1 à 6, à l'état de base, de sel, d'hydrate ou de solvat pharmaceutiquement acceptables, pour son utilisation comme médicament.

**10.** Utilisation d'un composé de formule (I) selon l'une quelconque des revendications 1 à 6, à l'état de base, de sel, d'hydrate ou de solvat pharmaceutiquement acceptable, pour la préparation d'un médicament destiné à traiter une pathologie dans laquelle un inhibiteur de la formation du peptide β-amyloïde β-A4 apporte un bénéfice thérapeutique.

**11.** Utilisation d'un composé de formule (I) selon l'une quelconque des revendications 1 à 6, à l'état de base, de sel, d'hydrate ou de solvat pharmaceutiquement acceptable, pour la préparation d'un médicament destiné à traiter la démence sénile, la maladie d'Alzheimer, le syndrome de Down, la maladie de Parkinson, l'angiopathie amyloïde, les désordres cérébro-vasculaires, les démences fronto-temporales et la maladie de Pick, les démences post-traumatiques, les pathologies liées à des processus de neuro-inflamation, la maladie de Huntington et/ou le syndrome de Korsakov.

**Claims**

**1.** Compound corresponding to the general formula (I):

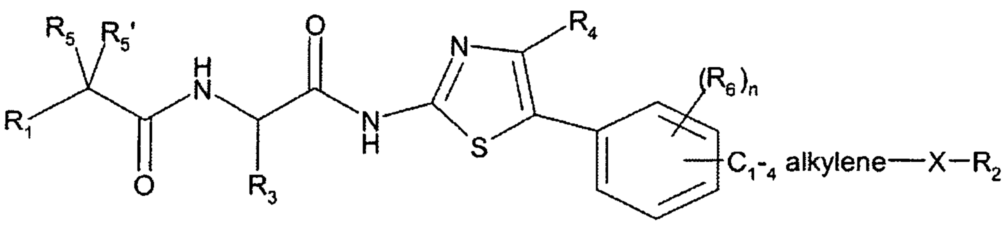

(I)

in which,
X represents an oxygen or sulphur atom;
$R_1$ represents, a $C_{1-10}$ alkyl group optionally substituted with a $C_{3-7}$ cycloalkyl, a phenyl or a thienyl; or $R_1$ represents a $C_{3-7}$ cycloalkyl, thienyl, pyridinyl or pyrimindinyl group;
the thienyl groups being optionally substituted with one to 3 $C_{1-3}$ alkyl groups; the phenyl group being optionally substituted with one to 5 halogen atoms or $C_{1-3}$ alkyl, $C_{1-3}$ alkoxy, $C_{1-3}$ fluoroalkyl or $C_{1-3}$ fluoroalkoxy groups;
$R_2$ represents a $C_{1-6}$ alkyl group optionally substituted with a $C_{3-7}$ cycloalkyl, phenyl, $C_{1-3}$ alkoxy, hydroxyl group; or $R_2$ represents a $C_{3-7}$ cycloalkyl, piperidinyl, phenyl or pyridinyl group;
the $C_{3-7}$ cycloalkyl and piperidinyl groups being optionally substituted with one or more $C_{1-3}$ alkyl, $C_{1-3}$ alkoxy, hydroxyl, $C_{1-3}$ fluoroalkyl or $C_{1-3}$ fluoroalkoxy groups;
the phenyl and pyridinyl groups being optionally substituted with one or more halogen atoms or $C_{1-3}$ alkyl, $C_{1-3}$ alkoxy, hydroxyl, $C_{1-3}$ fluoroalkyl or $C_{1-3}$ fluoroalkoxy;
$R_3$ represents a hydrogen atom or a $C_{1-6}$ alkyl group optionally substituted with a $C_{3-7}$ cycloalkyl group;
$R_4$ represents a hydrogen atom or a $C_{1-6}$ alkyl group; $R_5$ and $R_{5'}$ represent, independently of each other, a hydrogen or halogen atom, a hydroxyl or $C_{1-3}$ alkyl group; or $R_5$ and $R_{5'}$ form together an oxo or oxime group such as:

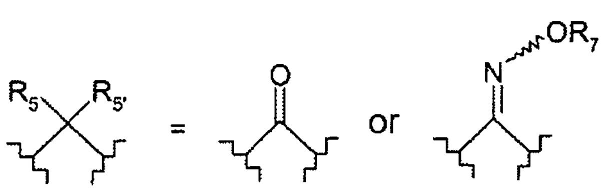

where $R_7$ represents a hydrogen atom or a $C_{1-3}$ alkyl; n represents an integer ranging from 0 to 3; and
$R_6$ represents independently of each other when n = 2 or 3, a hydrogen or halogen atom, a hydroxyl, $C_{1-3}$ alkyl, $C_{1-3}$ alkoxy, $C_{1-3}$ fluoroalkyl or $C_{1-3}$ fluoroalkoxy group;

in the form of a base, of an addition salt with an acid, of a hydrate or of a solvate.

**2.** Compound corresponding to the general formula (I):

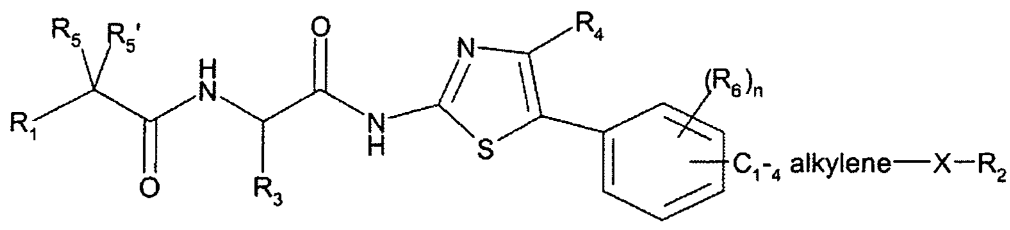

(I)

in which X, $R_1$, $R_3$, $R_4$, $R_5$, $R'_5$ and $R_6$ are as defined in Claim 1 and $R_2$ represents a $C_{1-6}$ alkyl group substituted with a phenyl group or $R_2$ represents a phenyl or pyridinyl group, the said phenyl and pyridinyl groups being substituted with one or more CN groups.

**3.** Compound of formula (I) according to Claim 1, **characterized in that:**

X represents an oxygen or sulphur atom;
$R_1$ represents a $C_{1-5}$ alkyl group, optionally substituted with a phenyl or a thienyl; or $R_1$ represents a $C_{3-7}$ cycloalkyl group, a thienyl or pyridinyl group; the thienyl groups being optionally substituted with one or two $C_{1-3}$ alkyl groups; the phenyl group being optionally substituted with one or two halogen atoms;
$R_2$ represents a $C_{1-6}$ alkyl, $C_{3-7}$ cycloalkyl, phenyl or pyridinyl group;
the phenyl group being optionally substituted with one to three $C_{1-3}$ alkyl, $C_{1-3}$ alkoxy, hydroxyl or fluoroalkoxy groups, or halogen atoms;
$R_3$ represents a $C_{1-6}$ alkyl group;
$R_4$ represents a hydrogen atom or a $C_{1-6}$ alkyl group;
$R_5$ and $R_{5'}$ represent, independently of each other, a hydrogen atom or a hydroxyl; or $R_5$ and $R_{5'}$ form together an oxo group;
$R_6$ represents a hydrogen or halogen atom, a $C_{1-3}$ alkyl, a $C_{1-3}$ alkoxy; and
n is 0 or 1;
in the form of a base, of an addition salt with an acid, of a hydrate or of a solvate.

**4.** Compound according to Claim 2, **characterized in that** $R_2$ represents a phenyl group, the said phenyl group being substituted with one to three CN groups, and X, $R_1$, $R_3$, $R_4$, $R_5$, $R'_5$ and $R_6$ are as defined in Claim 3.

**5.** Compound of formula (I) according to Claim 1 or 3, **characterized in that:**

X represents an oxygen atom;
$R_1$ represents a methyl, ethyl, 1-methylethyl, 1,1-dimethylethyl, propyl, 1-methylpropyl, 2-methylpropyl or 1-ethylpropyl, optionally substituted with a phenyl or with a thienyl; or $R_1$ represents a cyclohexyl, thienyl or pyridinyl group; the thienyl groups being optionally substituted with one or two methyl groups; the phenyl group being optionally substituted with one or two chlorine or fluorine atoms;
$R_2$ represents an ethyl, 1-methylethyl, cyclohexyl, phenyl or pyridinyl group;
the phenyl group being optionally substituted with one to three methyl, ethyl, methoxy, ethoxy, hydroxyl or trifluoromethoxy groups, or chlorine or fluorine atoms;
$R_3$ represents a methyl, ethyl or propyl group;
$R_4$ represents a hydrogen atom or a methyl or 4-methylpentyl group;
$R_5$ and $R_{5'}$ represent, independently of each other, a hydrogen atom, a hydroxyl; or $R_5$ and $R_{5'}$ form together an oxo group;
$R_6$ represents a hydrogen, chlorine or fluorine atom, a methyl, a methoxy or an ethoxy;
n is 0 or 1; and
the $C_{1-4}$ alkylene group is a methylene;

in the form of a base, of an addition salt with an acid, of a hydrate or of a solvate.

**6.** Compound according to Claim 2 or 4, **charcterized in that** $R_2$ represents a phenyl group, the said phenyl group being substituted with one to three CN groups and X, $R_1$, $R_3$, $R_4$, $R_5$, $R'_5$ and $R_6$ are as defined in Claim 5.

**7.** Method for preparing a compound of formula (I) according to any one of Claims 1 to 6, by peptide coupling of an amine of formula (XI)

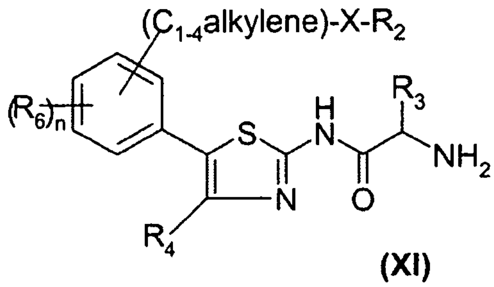

**(XI)**

with an acid of formula (XII)

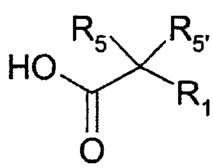

**(XII)**

in which X, $R_1$, $R_2$, $R_3$, $R_4$, $R_5$, $R_{5'}$, $R_6$ and n are as defined in formula (I) according to Claim 1.

**8.** Pharmaceutical composition containing at least one compound of formula (I) according to any one of Claims 1 to 6, in the form of a pharmaceutically acceptable base, salt, hydrate or solvate and optionally one or more pharmaceutically acceptable excipients.

**9.** Compound of formula (I) according to any one of Claims 1 to 6, in the form of a pharmaceutically acceptable base, salt, hydrate or solvate, for its use as a medicament.

**10.** Use of a compound of formula (I) according to any one of Claims 1 to 6, in the form of a pharmaceutically acceptable base, salt, hydrate or solvate, for the preparation of a medicament for treating a pathology in which an inhibitor of the formation of β-amyloid peptide β-A4 offers a therapeutic benefit.

**11.** Use of a compound of formula (I) according to any one of Claims 1 to 6, in the form of a pharmaceutically acceptable base, salt, hydrate or solvate, for the preparation of a medicament for treating senile dementia, Alzheimer's disease, Down's syndrome, Parkinson's disease, amyloid angiopathy, cerebrovascular disorders, frontotem-poral dementia and Pick's disease, post-traumatic dementia, pathologies linked to neuroinflammation processes, Huntington's disease and/or Korsakov's syndrome.

**Patentansprüche**

**1.** Verbindung der allgemeinen Formel (I):

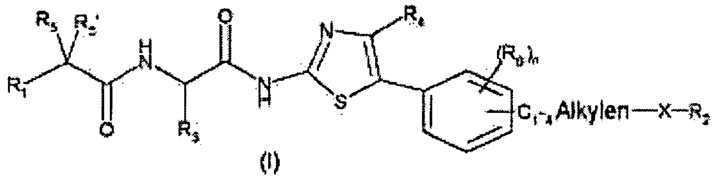

(I)

worin
X ein Sauerstoff- oder Schwefelatom darstellt;
$R_1$ eine $C_{1-10}$-Alkylgruppe darstellt, die gegebenenfalls mit einem $C_{3-7}$-Cycloalkyl, einem Phenyl, einem Thienyl substituiert ist; oder $R_1$ eine $C_{3-7}$-Cycloalkyl-, Thienyl-, Pyridinyl-, Pyrimidinylgruppe darstellt;
wobei die Thienylgruppen gegebenenfalls mit einer bis 3 $C_{1-3}$-Alkylgruppen substituiert sind; die Phenylgruppe gegebenenfalls mit ein bis 5 Halogenatomen oder $C_{1-3}$-Alkyl-, $C_{1-3}$-Alkoxy-, $C_{1-3}$-Fluoralkyl-, $C_{1-3}$-Fluoralk-oxygruppen substituiert ist;
$R_2$ eine $C_{1-6}$-Alkylgruppe darstellt, die gegebenenfalls mit einer $C_{3-7}$-Cycloalkyl-, Phenyl-, $C_{1-3}$-Alkoxy-, Hydroxygruppe substituiert ist; oder $R_2$ eine $C_{3-7}$-Cycloalkyl-, Piperidinyl-, Phenyl- oder Pyridinylgruppe darstellt;
wobei die $C_{3-7}$-Cycloalkyl- und Piperidinylgruppen gegebenenfalls mit einer oder mehreren $C_{1-3}$-Alkyl-, $C_{1-3}$-Alkoxy-, Hydroxy-, $C_{1-3}$-Fluoralkyl- oder $C_{1-3}$-Fluoralk-oxygruppen substituiert sind;
die Phenyl- und Pyridinylgruppen gegebenenfalls mit ein oder mehreren Halogenatomen oder $C_{1-3}$-Alkyl-, $C_{1-3}$-Alkoxy-, Hydroxy-, $C_{1-3}$-Fluoralkyl-, $C_{1-3}$-Fluoralkoxy-gruppen substituiert sind;
$R_3$ ein Wasserstoffatom oder eine $C_{1-6}$-Alkylgruppe darstellt, die gegebenenfalls mit einer $C_{3-7}$-Cycloalkylgruppe substituiert ist;
$R_4$ ein Wasserstoffatom oder eine $C_{1-6}$-Alkylgruppe darstellt;
$R_5$ und $R_{5'}$ unabhängig voneinander ein Wasserstoff-oder Halogenatom, eine Hydroxy-, $C_{1-3}$-Alkylgruppe darstellen; oder $R_5$ und $R_{5'}$ zusammen eine Oxo- oder Oximgruppe bilden, wie:

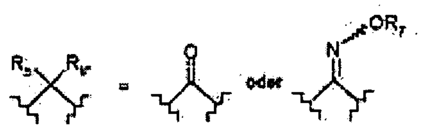

wobei $R_7$ ein Wasserstoffatom oder ein $C_{1-3}$-Alkyl darstellt;
n eine ganze Zahl von 0 bis 3 darstellt; und
$R_6$ unabhängig voneinander, wenn n = 2 oder 3, ein Wasserstoff-, Halogenatom, eine Hydroxy-, $C_{1-3}$-Alkyl-, $C_{1-3}$-Alkoxy-, $C_{1-3}$-Fluoralkyl- oder $C_{1-3}$-Fluoralkoxygruppe darstellt;
in Form einer Base, eines Additionssalzes an eine Säure, eines Hydrats oder Solvats.

2. Verbindung der allgemeinen Formel (I):

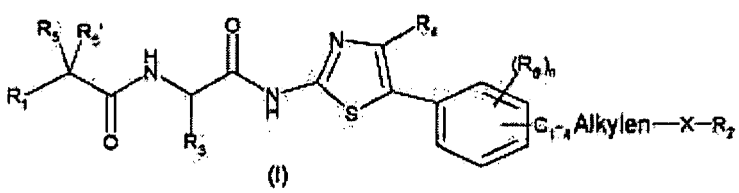

(I)

worin X, $R_1$, $R_3$, $R_4$, $R_5$, $R_{5'}$ und $R_6$ die im Anspruch 1 definierte Bedeutung haben und $R_2$ eine $C_{1-6}$-Alkylgruppe darstellt, die mit einer Phenylgruppe substituiert ist, oder $R_2$ eine Phenyl- oder Pyridinylgruppe darstellt, wobei diese Phenyl- und Pyridinylgruppen mit einer oder mehreren CN-Gruppen substituiert sind.

3. Verbindung der Formel (I) nach Anspruch 1, **dadurch gekennzeichnet, dass:**

X ein Sauerstoff- oder Schwefelatom darstellt;
$R_1$ eine $C_{1-5}$-Alkylgruppe darstellt, die gegebenenfalls mit einem Phenyl, einem Thienyl substituiert ist; oder $R_1$ eine $C_{3-7}$-Cycloalkylgruppe, eine Thienyl-, Pyridinylgruppe darstellt; wobei die Thienylgruppen gegebenenfalls mit einer oder zwei $C_{1-3}$-Alkylgruppen substituiert sind; die Phenylgruppe gegebenenfalls mit ein oder zwei Halogenatomen substituiert ist;
$R_2$ eine $C_{1-6}$-Alkyl-, $C_{3-7}$-Cycloalkyl-, Phenyl- oder Pyridinylgruppe darstellt;
wobei die Phenylgruppe gegebenenfalls mit ein bis drei $C_{1-3}$-Alkyl-, $C_{1-3}$-Alkoxy-, Hydroxy-, Fluoralkoxygruppen oder Halogenatomen substituiert ist;
$R_3$ eine $C_{1-6}$-Alkylgruppe darstellt;
$R_4$ ein Wasserstoffatom oder eine $C_{1-6}$-Alkylgruppe darstellt;
$R_5$ und $R_{5'}$ unabhängig voneinander ein Wasserstoffatom, ein Hydroxy darstellen; oder $R_5$ und $R_{5'}$ zusammen eine Oxogruppe bilden;
$R_6$ ein Wasserstoff-, ein Halogenatom, ein $C_{1-3}$-Alkyl, $C_{1-3}$-Alkoxy darstellt; und
n gleich 0 oder 1 ist;
in Form einer Base, eines Additionssalzes an eine Säure, eines Hydrats oder Solvats.

**4.** Verbindung nach Anspruch 2, **dadurch gekennzeichnet, dass** $R_2$ eine Phenylgruppe darstellt, wobei die Phenylgruppe mit einer bis drei CN-Gruppen substituiert ist und X, $R_1$, $R_3$, $R_4$, $R_5$, $R_{5'}$ und $R_6$ die in Anspruch 3 definierte Bedeutung haben.

**5.** Verbindung der Formel (I) nach Anspruch 1 oder 3, **dadurch gekennzeichnet, dass:**

X ein Sauerstoffatom darstellt;
$R_1$ ein Methyl, Ethyl, 1-Methylethyl, 1,1-Dimethylethyl, Propyl, 1-Methylpropyl, 2-Methylpropyl oder 1-Ethylpropyl darstellt, das gegebenenfalls mit einem Phenyl oder mit einem Thienyl substituiert ist; oder $R_1$ ein Cyclohexyl, Thienyl oder Pyridinyl darstellt; wobei die Thienylgruppen gegebenenfalls mit einer oder zwei Methylgruppen substituiert sind; die Phenylgruppe gegebenenfalls mit einem oder zwei Chlor-oder Fluoratomen substituiert ist;
$R_2$ eine Ethyl-, 1-Methylethyl-, Cyclohexyl-, Phenyl-oder Pyridinylgruppe darstellt;
wobei die Phenylgruppe gegebenenfalls mit ein bis drei Methyl-, Ethyl-, Methoxy-, Ethoxy-, Hydroxy-, Trifluormethoxygruppen oder Chlor- oder Fluoratomen substituiert ist;
$R_3$ eine Methyl-, Ethyl- oder Propylgruppe darstellt;
$R_4$ ein Wasserstoffatom oder eine Methyl- oder 4-Methylpentylgruppe darstellt;
$R_5$ und $R_{5'}$ unabhängig voneinander ein Wasserstoffatom, ein Hydroxy darstellen; oder $R_5$ und $R_{5'}$ zusammen eine Oxogruppe bilden;
$R_6$ ein Wasserstoff-, Chlor- oder Fluoratom, ein Methyl, ein Methoxy oder ein Ethoxy darstellt;
n gleich 0 oder 1 ist; und
die $C_{1-4}$-Alkylengruppe ein Methylen ist;
in Form einer Base, eines Additionssalzes an eine Säure, eines Hydrats oder Solvats.

**6.** Verbindung nach Anspruch 2 oder 4, **dadurch gekennzeichnet, dass** $R_2$ eine Phenylgruppe darstellt, wobei die Phenylgruppe mit einer bis drei CN-Gruppen substituiert ist und X, $R_1$, $R_3$, $R_4$, $R_5$, $R_{5'}$ und $R_6$ die im Anspruch 5 definierte Bedeutung haben.

**7.** Verfahren zur Herstellung einer Verbindung der Formel (I) nach einem der Ansprüche 1 bis 6, durch Peptidkopplung eines Amins der Formel (XI)

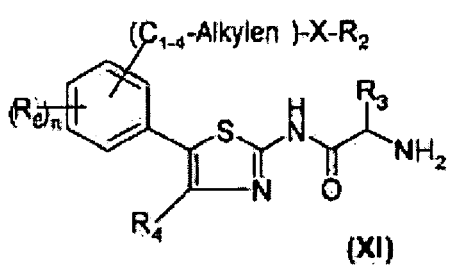

**(XI)**

mit einer Säure der Formel (XII)

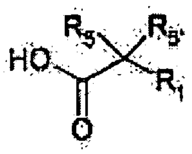 (XII)

worin X, $R_1$, $R_2$, $R_3$, $R_4$, $R_5$, $R_{5'}$, $R_6$ und n die in der Formel (I) gemäß Anspruch 1 definierte Bedeutung haben.

**8.** Pharmazeutische Zusammensetzung, die mindestens eine Verbindung der Formel (I) nach einem der Ansprüche 1 bis 6 in Form einer pharmazeutisch annehmbaren Base, eines pharmazeutisch annehmbaren Salzes, Hydrats oder Solvats und einen oder mehrere pharmazeutisch annehmbare Excipienten enthält.

**9.** Verbindung der Formel (I) nach einem der Ansprüche 1 bis 6 in Form einer pharmazeutisch annehmbaren Base, eines pharmazeutisch annehmbaren Salzes, Hydrats oder Solvats zur Verwendung als Arzneimittel.

**10.** Verwendung einer Verbindung der Formel (I) nach einem der Ansprüche 1 bis 6 in Form einer pharmazeutisch annehmbaren Base, eines pharmazeutisch annehmbaren Salzes, Hydrats oder Solvats zur Herstellung eines Arzneimittels zur Behandlung einer Pathologie, bei der ein Inhibitor der Bildung des β-Amyloidpeptids β-A4 einen therapeutischen Nutzen erbringt.

**11.** Verwendung einer Verbindung der Formel (I) nach einem der Ansprüche 1 bis 6 in Form einer pharmazeutisch annehmbaren Base, eines pharmazeutisch annehmbaren Salzes, Hydrats oder Solvats zur Herstellung eines Arzneimittels zur Behandlung von seniler Demenz, Alzheimer-Krankheit, Down-Syndrom, Parkinson-Krankheit, Amyloid-Angiopathie, zerebrovaskulären Störungen, frontotemporalen Demenzen und Pick-Krankheit, posttraumatischen Demenzen, Pathologien in Verbindung mit neuroinflammatorischen Vorgängen, Huntington-Krankheit und/oder Korsakov-Syndrom.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 03014095 A
- WO 9828268 A

### Littérature non-brevet citée dans la description


- **J. MARCH.** Advanced Organic Chemistry. Wiley Interscience, 310-316
- **GREENE et al.** Protective groups in Organic Synthesis. John Wiley & Sons, Inc,
- **SCHOEVAARS.** *J. Am. Chem. Soc.,* 1999, vol. 121, 9550-9561
- **WOLFE.** *J. Org. Chem.,* 1997, vol. 62, 4943-4948
- **KAYE.** *J. Chem. Soc. Perkins I,* 1981, 2335-2339
- **MIDDLETON et al.** *J. Org. Chem.,* 1980, vol. 45 (14), 2883-2887
- **MIYAMOTO et al.** *J. Amer. Chem. Soc.,* 1992, vol. 114 (15), 6256-6257
- **WOLFE MS.** Secretase targets for Alzheimer's disease: identification and therapeutic potential. *J. Med. Chem.,* 21 Juin 2001, vol. 44 (13), 2039-60
- **WOLFE MS ; HAASS C.** The Role of presenilins in gamma-secretase activity. *J. Biol. Chem.,* 23 Février 2001, vol. 276 (8), 5413-6